(19) Europäisches Patentamt
European Patent Office
Office européen des brevets

(11) Publication number: **0 112 511**
**B1**

(12) # EUROPEAN PATENT SPECIFICATION

(45) Date of publication of patent specification: **06.09.89**

(51) Int. Cl.$^4$: **C 07 K 5/06,** A 61 K 37/02

(21) Application number: **83111894.8**

(22) Date of filing: **28.11.83**

(54) **New compounds, pharmaceutical preparations containing same and process for their preparation.**

(30) Priority: **29.11.82 US 445117**

(43) Date of publication of application:
**04.07.84 Bulletin 84/27**

(45) Publication of the grant of the patent:
**06.09.89 Bulletin 89/36**

(84) Designated Contracting States:
**AT BE CH DE FR GB IT LI LU NL SE**

(56) References cited:
**EP-A-0 061 768**
**EP-A-0 121 830**
**US-A-4 374 829**

(73) Proprietor: **RORER INTERNATIONAL (OVERSEAS) INC.**
**P.O. Box 145**
**Lewes, DE 19958 (US)**

(72) Inventor: **Fu-Chih, Huang**
**611 Knoll Road**
**Boonton, N.J. (US)**
Inventor: **Jones, Howard**
**79 Briarcliff Woods**
**Ossining, N.Y. (US)**
Inventor: **Wan-Kit, Chan**
**Parkway Drive**
**Yorktown Heights, N.Y. (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

**EP 0 112 511 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This application relates to compounds of the general formula I

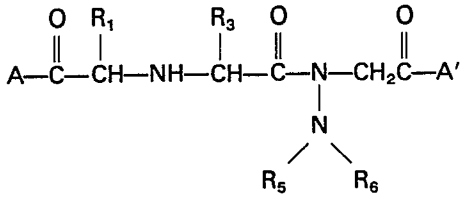

(I)

and their pharmaceutically acceptable salts wherein

A and A′ are independently hydroxy or $C_1$—$C_6$-alkoxy;

$R_1$ is $C_1$—$C_9$-alkyl, arylalkyl or aryloxyalkyl with 1 to 9 carbon atoms in the alkyl moiety; and

$R_3$ is $C_1$—$C_6$-alkyl, or ω-amino $C_1$—$C_6$-alkyl,

$R_5$ and $R_6$ are independently hydrogen, $C_1$—$C_9$-alkyl aryl, aralkyl with 1 to 9 carbon atoms in the alkyl moiety or pyridyl, or

$R_5$ and $R_6$ when taken together with the nitrogen atom to which they are attached form a substituted or unsubstituted, saturated or unsaturated monocyclic or polycyclic ring containing from 2 to 9 carbon atoms which may include one oxygen, sulfur or additional nitrogen atom.

Said compounds possess biological activity as inhibitors of the enzymatic conversion of angiotensin I to angiotensin II. As such, the compounds of the present invention have utility in the treatment of hypertension in subjects suffering therefrom.

EP—A—0061768 discloses N-amido substituted dipeptides possessing hypertensive and angiotensin converting enzyme inhibitory activity.

EP—A—0121830 discloses substituted dipeptides having antihypertensive activity.

US—A—4374829 discloses carboxyalkyl dipeptide derivatives which are useful as antihypertensives.

In the compounds of the present invention the ring which may be formed by $R_5$ and $R_6$ can be substituted with hydroxy, alkyl, alkoxy, aryl, aryloxy, aralkyl, or mono- or dialkylamino, and can be fused with another aryl or cycloalkyl ring which can be substituted with hydroxy, alkyl, alkoxy, aryl, aryloxy, aralkyl, or mono- or dialkylamino.

The cycloalkyl rings where not fused with both $R_5$ and $R_6$, can be substituted with alkyl, halo, haloalkyl, hydroxy, alkylamino, or nitro groups; and the aryl rings, where not fused with both $R_5$ and $R_6$, may be substituted with carboxylic acid, cyano, carbo-lower alkoxy, alkyl, hydroxy, alkoxy, hydroxyalkyl, halo, haloalkyl, thio, alkylmercapto, thioalkyl, amino, alkylamino, aminoalkyl, nitro, methylenedioxy or sulfonylamino groups.

Preferred compounds of the present invention are those wherein $R_5$ and $R_6$ are independently hydrogen, $C_1$—$C_9$-alkyl or aryl or are joined together to form an alkylene or hetero-alkylene bridge or fused aralkylene, in which the alkyl, aryl, cycloalkyl bridge, and fused aralkylene can be substituted or unsubstituted. Included as preferred groups are groups in which $R_5$ or $R_6$ provide diuretic activity to the compound of the formula I, e.g., sulfonamido-chloro-phenyl.

The alkyl groups per se and the alkyl moieties in alkoxy, aralkyl, and aminoalkyl may be straight-chained or branched. Such groups include, for example, methyl, ethyl, propyl, isopropyl, butyl, isobutyl, tertiary-butyl, amyl, iso-amyl, hexyl and octyl. Preferably the alkyl groups contain from 1 to 6 carbon atoms, straight-chained or branched.

The preferred substituents on the above alkyl groups include hydroxy, alkoxy, amino, alkylamino, dialkylamino, mercapto, alkylmercapto and halogen.

Preferred cyclic and polycyclic ring structures include such radicals as cyclopropyl, cyclobutyl, cyclopentyl, cyclohexyl, cycloheptyl, adamantyl, norbornyl, phenyl, tolyl, benzyl, phenethyl, indolyl, dimethoxyphenyl, hydroxybenzyl, indanyl, naphthyl, tetrahydronaphthyl, decanhydronaphthyl, pyridyl, quinolyl, guanidino, pyrrolidyl, pyrrolyl, morpholinyl, furyl, furfuryl, tetrahydrofurfuryl, benzimidazolyl, thienyl and imidazolyl.

Of these, the structures containing at least one nitrogen atom are preferred structures for those embodiments in which $R_5$ and $R_6$ are connected to each other. Preferred substituents on the ring structures which can be formed by connecting $R_5$ and $R_6$ include hydroxy, alkyl, alkoxy, aryl, aryloxy, aralkyl, alkylamino, and dialkylamino; these and alkenyl, alkynyl, carboxy, carboalkoxy, cyano, mercapto, amino, alkylmercapto, halo, trifluoromethyl and sulfonamide, are preferred substituents on all other aryl, cycloalkyl, fused arylcycloalkyl, and polycycloalkyl ring structures.

The halo groups include fluoro, chloro, bromo and iodo.

Substituents which are "unsaturated" contain one or more double or triple bonds.

The compounds of the present invention may contain one or more asymmetric carbon atoms and may exist in racemic and optically active forms. All of these forms are contemplated to be within the scope of the present invention.

The compounds of the present invention are prepared by amide-forming reaction of a compound of formula II

$$R_5R_6N—NH—CH_2COR_9 \qquad \text{II}$$

with an acylating derivative of an acid of formula III

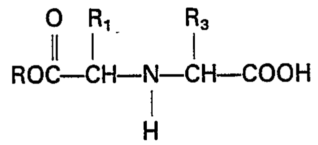

III

wherein $R_1$, $R_3$, $R_5$ and $R_6$ are as defined above and R and $R_9$ are hydrogen or lower alkyl, to give the desired compound.

As an alternative approach, a dipeptide of formula IV

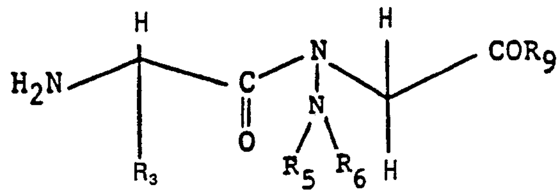

IV

is reacted with an α keto-acid or ester of formula V

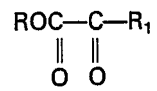

V

wherein R, $R_1$, $R_3$, $R_5$, $R_6$ and $R_9$ are as defined above, to form the corresponding imine and the imine is reduced to give a compound of formula I in which $R_3$ is H.

As a further reaction, the compounds of the present invention can be prepared by reaction of a compound of formula VI

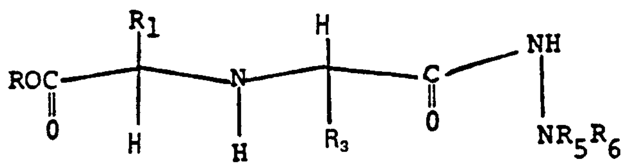

VI

with an α-halo compound of formula VII

$$Hal—CH_2—COR_9 \qquad \text{VII}$$

wherein R, $R_1$, $R_3$, $R_5$, $R_6$ and $R_9$ are as defined above and Hal is halogen by cleavage of hydrogen halide.

The aforementioned condensation reaction to form imines with subsequent hydrogenation can be conveniently carried out in a single reaction zone by the expediency of mixing the α-keto acid or derivative with the reactive compound of formula IV under hydrogenation conditions. For practical purposes, the aforesaid reactants can be hydrogenated over noble metal catalyst such as palladium, platinum, rhodium or ruthenium, and the two stages occur under such conditions to produce the desired end-product.

As in any organic reaction solvents can be employed such as methanol, ethanol, propanol, acetone, tetrahydrofuran, dioxane, dimethylformamide, or diethylacetamide.

The reaction is normally effected at or near room temperature, although temperatures from 0°C up to the reflux temperature of the reaction mixture can be employed.

Preferably, R and $R_9$ are hydrogen or lower alkyl, $R_5$ is hydrogen, and $R_1$ is $C_1$—$C_9$-alkyl.

The amide forming conditions referred to herein involve the use of known derivatives of the described acids, such as the acyl halides, anhydrides, mixed anhydrides, lower alkyl esters, carbodiimides or carbonyl diimidazoles.

The reactions are carried out in organic solvents such as acetonitrile, tetrahydrofuran, dioxane, acetic acid, methylene chloride or ethylene chloride.

The amide forming reaction will occur at room temperature or at elevated temperature. The use of elevated temperature is for convenience in that it permits somewhat shortened reaction periods. Temperatures ranging from 0°C up to the reflux temperature of the reaction system can be used. As a

further convenience the amide forming reaction can be effected in the presence of a base such as tertiary organic amines, e.g., trimethylamine, pyridine or picolines, particularly where hydrogen halide is formed by the amide-forming reaction, e.g., acyl halide and amino compound. Of course, in those reactions where hydrogen halide is produced, any of commonly used hydrogen halide acceptors can also be used.

In the condensation of an alpha haloacid derivative of formula VII similar reaction conditions, solvents and hydrogen halide acceptors can be used as for amide formation.

Various substituents on the compounds of the present invention can be present in the starting compounds or added after formation of the amide products by known methods of substitution or conversion reactions. Thus, the nitro group can be added to the final product by nitration of the aromatic ring and the nitro group converted to other groups, such as amino by reduction, and halo by diazotization of the amino groups and replacement of the diazo group. Other reactions can be effected on the formed amide product. Amino groups can be alkylated to form mono and dialkylamino groups, mercapto and hydroxy groups can be alkylated to form corresponding ethers. Thus, substitution or alteration reactions can be employed to provide a variety of substituents throughout the molecule of the final products. Of course, reactive groups where present should be protected by suitable blocking groups during any of the aforesaid reactions particularly the condensation reactions to form the amide linkages.

The acid and base salts of the compounds of the present invention can be formed using standard procedures. Often, they are formed in situ during the preparation of the present new amido amino acids.

The present compounds obviously exist in stereoisomeric forms and the products obtained thus can be mixtures of the isomers, which can be resolved. Alternatively, by selection of specific isomers as starting compounds, the preferred stereoisomer can be produced. Therefore, the preferred forms, where each asymmetric center (chiral center) is S-configuration, are preferably prepared by stereospecific route rather than attempting resolution of mixtures of isomers. The compounds in which the S-configuration exists at all asymmetric centers are the most active; those in which the R-configuration exists are of less activity; and those where both R- and S-configurations exist are of intermediate activity.

The products are obtained typically as a mixture of diastereoisomers which can be separated by standard methods of fractional crystallization or chromatography.

The compounds of this invention form acid salts with various inorganic and organic acids which are also within the scope of the invention. The pharmaceutically acceptable acid addition salts of the compounds of the present invention may be prepared by conventional reactions by reacting the free amino acid or amino ester with an appropriate acid providing the desired anion, either in a solvent or medium in which the salt is insoluble, or in water and removing the water by freeze-drying. The salts of strong acids are preferred. Exemplary pharmaceutically acceptable acid salts are the salts of hydrochloric, hydrobromic, sulfuric, nitric, acetic, furmaric, malic, maleic and citric acids.

Suitable basic salts may include the salts of alkali and alkali earth metals such as sodium, lithium, potassium, magnesium and calcium, as well as iron and salts of ammonia and amines, and quaternary ammonium salts.

The action of the enzyme renin or angiotensinogen, a pseudoglobulin in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds of this invention intervene in the renin -to- angiotensin I -to- angiotensin II sequence by inhibiting angiotensin I converting enzyme and reducing or eliminating the formation of the pressor substance angiotensin II and therefore are useful in reducing or relieving hypertension. Thus by the administration of a composition containing one or a combination of compounds of formula I or pharmaceutically acceptable salts thereof, hypertension in the species of mammal suffering therefrom is alleviated. A single dose, or preferably two to four divided daily doses, provided on a basis of about 0.1 to 100 mg per kg per day, preferably about 1 to 50 mg per kg per day, is appropriate to reduce blood pressure. The substance is preferably administered orally, but a parenteral route such as subcutaneously, intramuscularly, intravenously or intraperitonealy can also be employed.

When evaluated according to standard, recognized *in vivo* methods, a compound of the present invention in the (S,S) form and having the structure (5):

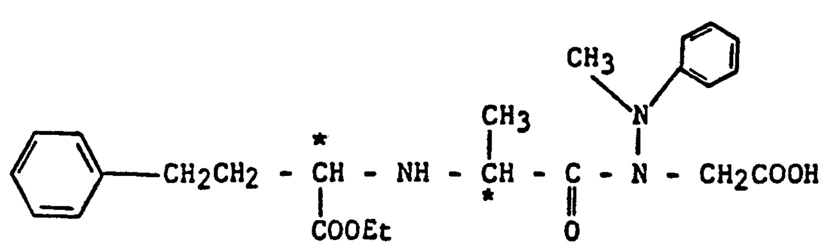

(5)

exhibited angiotensin converting enzyme inhibition of 52% and 79% at a dose level of 1 mg/kg. $ED_{50}$ values of less than 1 mg/kg have been exhibited by compounds (5) and (6):

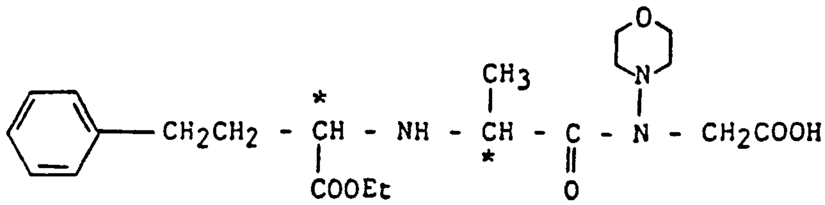

(6)

Additional compounds were also tested using both *in vitro* and *in vivo* standard procedures and the results are provided in the following table in which $I_{(50)}$ values are for *in vitro* and $ED_{(50)}$ values are for *in vivo* evaluations:

**Table 1**

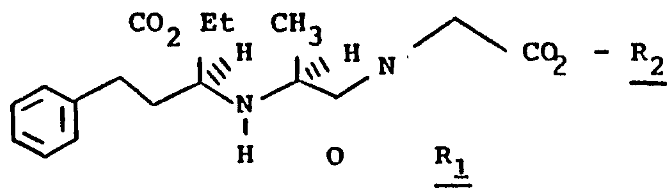

| | Isomer | M.P. (°C.) | $R_1$ | $R_2$ | $I_{(50)}$ | $ED_{(50)}$ |
|---|---|---|---|---|---|---|
| 1) | (SS) | --- | N-methylanilino | H | 2.1 μM | 0.3-1.0 mg/kg, p.o. |
| 2) | (SS) | 110 (2HCl) | 1-morpholinyl | H | 17 μM | 0.1 mg/kg, p.o. |
| 3) | (SS) | 60-64 | 1-(2-methylindolyl) | H | 15 μM | 0.3-3 mg/kg, p.o. |
| 4) | (SS) | --- | anilino | H | --- | 41 mg/kg, p.o. |
| 5) | (SS) | 118 (HCl) | N-methyl-4-chloroanilino | H | --- | 100 mg/kg, p.o. |
| 6) | (SS) | 165 (2HBr) | 1-morpholinyl | H | 9.5 μM | 3 mg/kg. p.o. |
| 7) | (SSS) | 110-118 (HCl) | 1-pyrrolyl | H | 66% 100 μM | 100 mg/kg, i.p.<br>3 mg/kg, p.o. |
| 8) | (SS) | 112-118 (HCl) | N-methyl-3-chloroanilino | H | 4.6 μM | 100 mg/kg, p.o.<br>3 mg/kg, p.o.<br>1 mg/kg, p.o. |
| 9) | (SS) | 104-109 (HCl) | N-methyl-2-chloroanilino | H | 85% 100 μM | 3 mg/kg, p.o. |

Table 1 (Continued)

| Isomer | M.P. (°C.) | $R_1$ | $R_2$ | $I_{(50)}$ | $ED_{(50)}$ |
|---|---|---|---|---|---|
| 10) (SS) | 136-141 (2HCl) | N-methyl-2-pyridylanino | H | --- | 3 mg/kg, p.o. |
| 11) (SS) | 113-118 (HCl) | N-methyl-2-chloroanilino | t-butyl | 88 µM | 3 mg/kg, p.o. |
| 12) (SS) | 92.8 (HCl) | N-methyl-3-chloroanilino | t-butyl | 4.5 µM | 3 mg/kg, p.o. |
| 13) (SS) | 133-138 (2HCl) | N-methyl-2-pyridylanilino | t-butyl | --- | 3 mg/kg, p.o. |
| 14) (SS) | 82.8 (HCl) | $-N-COO-CH_2-$benzyl | H | --- | 3 mg/kg, p.o. |
| 15) (SS) | 145-153 (2HCl) | 1-(4-methyl pyrazinyl) | H | --- | 3 mg/kg, p.o. |
| 16) (SS) | 146-149 (HCl) | $CH_3NH-$ | H | --- | 3 mg/kg, p.o. |
| 17) (SS) | 155-156 (HCl) | 1-pyrrolyl | H | --- | 3 mg/kg, p.o. |

The compounds of the invention can be utilized to achieve the reduction of blood pressure by formulating one or more of them in compositions such as tablets, capsules or elixirs for oral administration or in sterile solutions or suspensions for parenteral administration. About 10 to 500 mg of a compound or mixtures of compounds of formula (I) or pharmaceutically acceptable salt(s) thereof is compounded with a physiologically acceptable vehicle, carrier, excipient, binder, preservative, stabilizer or flavor, in a unit dosage form as called for by pharmaceutical practice. The amount of active substance in these compositions or preparations is such that a suitable dosage in the range indicated is obtained.

Illustrative of the adjuvants which may be incorporated, for example, in tablets and capsules are the following: a binder such as gum tragacanth, acacia, corn starch or gelatin; an excipient such as dicalcium phosphate; a disintegrating agent such as corn starch, potato starch or alginic acid; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil or cottonseed oil, or a synthetic fatty vehicle like ethyl oleate. Buffers, preservatives and antioxidants can be incorporated as required.

Specific embodiments of the invention are illustrated in the following Examples.

Preparation of intermediate product:

An intermediate employed in the following examples, N-[[1S)-ethoxycarbonyl-3-phenylpropyl]-N-(2,2,2-trichloroethoxy-carbonyl)-L-alanine acid chloride,

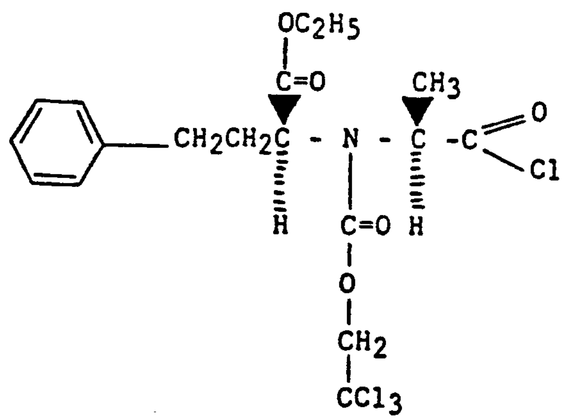

(I-D)

was prepared as follows:

To a mixture of N[(1S)-ethoxycarbonyl-3-phenylpropyl]-L-alanine (10 g) and sulfuric acid (10 ml) in 100 ml of dioxane was added 150 ml of isobutylene, and the resulting reaction mixture was shaken in a pressure bottle overnight. The reaction mixture was neutralized with 50% NaOH, taken up in 200 ml of ethyl acetate, and washed with water. The organic solution was dried and evaporated to dryness to give 10 g of oily product (I-A), N-[(1S)-Ethoxycarbonyl-3-phenylpropyl]-L-alanine t-butyl ester:

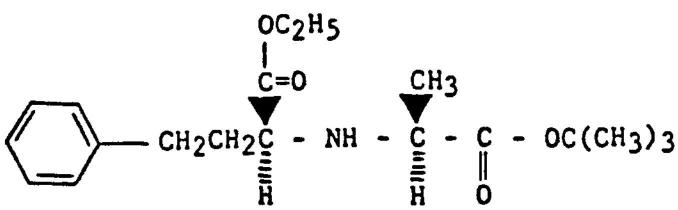

(I-A)

A mixture containing 2.52 g (7.51 mmol) of compound (I-A), 1.10 ml (7.99 mmol) of 2,2,2-trichloroethyl chloroformate, and 1.0 ml (12.4 mmol) of pyridine in 25 ml of dry tetrahydrofuran was refluxed under a nitrogen atmosphere for 3 h. The reaction mixture was filtered, taken up in 200 ml of ether, and washed four times with 1N hydrochloric acid and once with brine. The organic phase was dried over $MgSO_4$,

filtered, and concentrated in vacuo to yield 3.79 g (99%) of compound (I-B), N-[(1S)-1-ethoxycarbonyl-3-phenylpropyl]-N-(2,2,2-trichloroethoxycarbonyl)-L-alanine t-butyl ester:

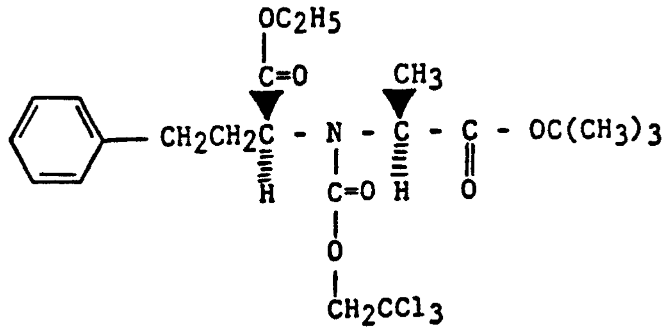

(I-B)

A mixture containing 1.98 g (3.88 mmol) of compound (I-B) in 25 ml of 4N HCl in dioxane at room temperature and under a nitrogen atmosphere was stirred for 8 h. The mixture was then concentrated in vacuo to provide 1.77 g (100%) of compound (I-C, N-[(1S)-1-ethoxycarbonyl-3-phenylpropyl)-N-(2,2,2-trichloroethoxycarbonyl)-L-alanine:

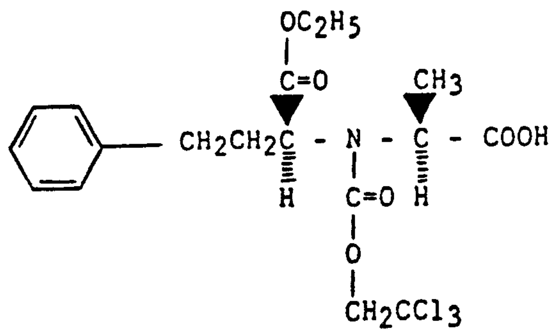

(I-C)

To a mixture containing 908 mg (2.00 mmol) of compound (I-C) and 0.40 ml (4.6 mmol) of oxalyl chloride in 10 ml of dry methylene chloride at room temperature and under a nitrogen atmosphere was added 10 l (0.13 mmol) of N,N-dimethylformamide. After 2 h the mixture was carefully concentrated in vacuo (T<30°C) to give 900 ml of the acid chloride, compound (I-D).

Example I

A mixture of 1-methyl-1-phenylhydrazine (5 g, 41 mmol) t-butyl bromoacetate (8 g, 41 mmol), and sodium carbonate (2.2 g, 21 mmol) in 200 ml of DMF was stirred at room temperature overnight. DMF was removed in vacuo. The residue was extracted into ethyl acetate and the organic solution was washed with water, dried and evaporated to give 6.5 g of crude oily product. Purification by dry column chromatography gave 2 g of t-butyl (N'-methyl-N'-phenyl)hydrazinoacetate (compound II-A). A solution of 1.11 g of compound (II-A) and 3 ml of pyridine in 10 ml of methylene chloride was added dropwise to a solution of 2 g of compound (I-D) in 10 ml of methylene chloride. The reaction mixture was stirred at room temperature overnight and was then washed with 1N hydrochloric acid, aqueous sodium bicarbonate, and brine. The organic solution was dried over magnesium sulfate, filtered, concentrated in vacuo, and purified by dry column chromatography to give 1.5 g of slightly impure desired product (II-B), N-(N-methylanilino)-N-[N-[(1S)-1(ethoxycarbonyl)-3-phenylpropyl]-N-(2,2,2-trichloroethoxycarbonyl)-L-alanyl]glycine t-butyl ester:

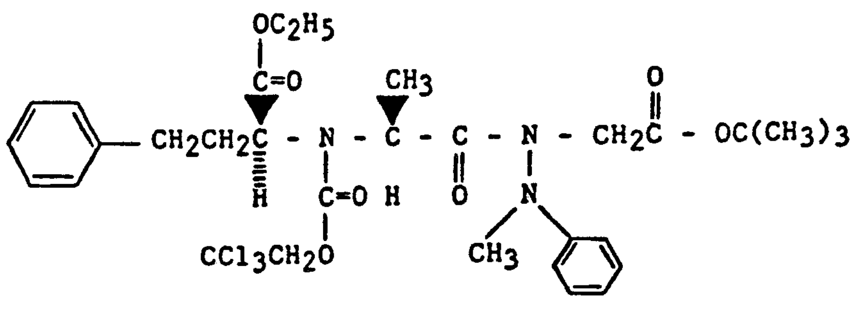

(II-B)

To a solution of 1.5 g of product (II-B) in 10 ml of glacial acetic acid was added 2.5 g of zinc dust. After 2 hours the mixture was filtered through Celite® and concentrated in vacuo. The residue was taken up in

ether and washed successively with sodium bicarbonate solution, water, and brine and dried. After filtration, the solution was concentrated in vacuo and purified by dry column chromatography to give 0.7 g of pure product (II-C), N-(N-methylanilino)-N-[N-[(1S)-1-ethoxycarbonyl-3-phenylpropyl]-L-alanyl]glycine t-butyl ester:

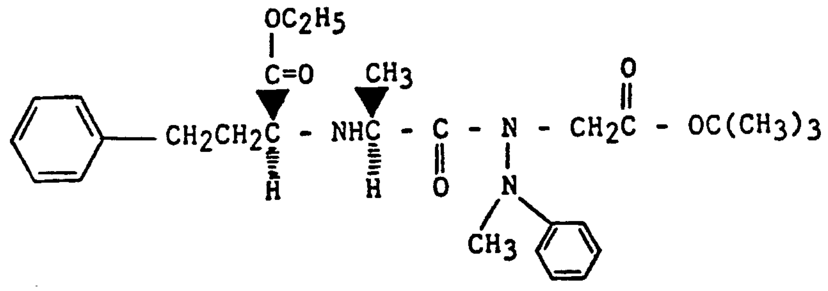

(II-C)

This product was converted to the dihydrochloric acid salt of the free acid by bubbling a stream of hydrogen chloride gas into an ethereal solution of 0.7 g of product (II-C) at 0°C for 3 h. The solution was concentrated in vacuo to give 0.4 g of white powder.

Example II

A mixture of N-aminomorpholine (4 g), t-butyl bromoacetate (7.6 g) and sodium carbonate (2.1 g) in 20 ml of DMF was stirred at room temperature overnight. After concentration in vacuo, the residue was taken up in ethyl acetate. The organic solution was washed with water, dried, filtered and concentrated in vacuo to give 2.5 g of crude N-(morpholino)glycine t-butyl ester (compound III-A). This crude compound was used without further purification. To a solution of 5 g of the product (I-D) in 10 ml of methylene chloride was added a solution of 2.5 g of compound (III—A) and 5 ml of pyridine in 5 ml of methylene chloride over a period of 10 min. After 16 h the reaction mixture was washed successively with 1N hydrochloric acid, saturated aqueous sodium bicarbonate, and once with brine. The organic solution was dried, filtered, concentrated in vacuo, and purified by dry column chromatography to give 3.5 g of oily product (III-B) N-(morpholino)-N-[N-[(1S)-1-ethoxycarbonyl-3-phenyl-propyl]-N-(2,2,2-trichloroethoxycarbonyl)-L-alanyl]glycine t-butyl ester:

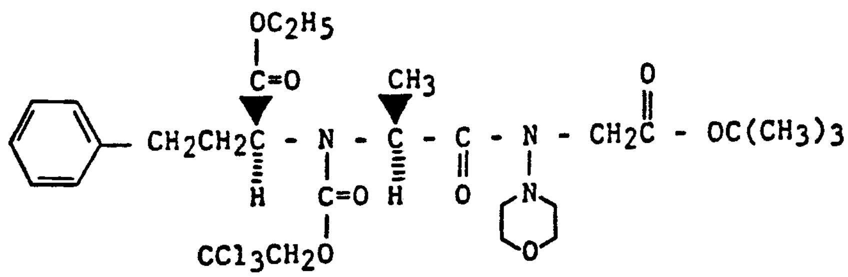

(III-B)

To a solution of 0.6 g of product (III-B) in 10 ml of glacial acetic acid was added 2 g of zinc dust. After 3 h the mixture was filtered through celite and concentrated in vacuo. The residue was taken up in ethyl acetate and washed with water. The organic solution was dried, filtered, concentrated in vacuo, and chromatographed to give 0.4 g of product (III-C), N-(morpholine)-N-[N-[(1S)-1-ethoxycarbonyl-3-phenyl-propyl]L-alanyl]glycine t-butyl ester:

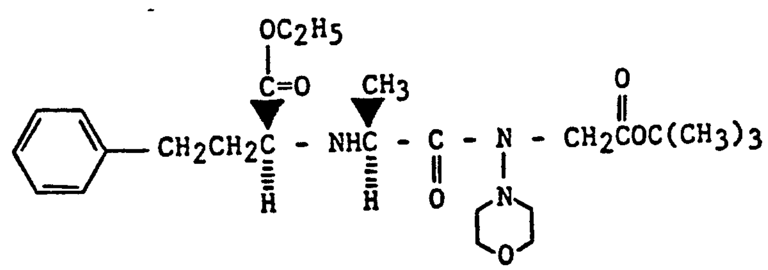

(III-C)

The dihydrochloric acid salt of the free acid was formed by bubbling dry hydrogen chloride gas into an etheral solution containing 0.5 g of product (III-C) at 0°C for 2.5 h. Concentration in vacuo gave 0.4 g of solid product.

Example III

A mixture of 1-amino-2-methylindoline (3.7 g), potassium carbonate (2.8 g) and t-butyl bromoacetate (4 g) in 10 ml of DMF was stirred at room temperature overnight: After removal of DMF in vacuo, the residue was taken up in ethyl acetate. The organic solution was washed with water, dried with magnesium sulfate, filtered, and concentrated. The product was purified by dry column chromatography to give 1.5 g of slightly impure oily product (IV-A), N-[1-(2-methyl-indolino)]glycine t-butyl ester. This was used for the next reaction without further purification.

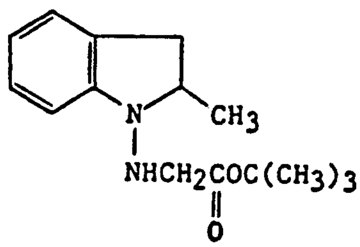

(IV-A)

A solution of 2.25 g of acid chloride product (I-D) in 10 ml of methylene chloride was added dropwise to a 15 ml of methylene chloride solution containing 1.5 g of product (IV-A) and 0.4 g of pyridine over a period of 10 min. After 20 h the reaction mixture was washed successively with 1N hydrochloric acid, saturated aqueous sodium bicarbonate and brine solution. The organic solution was dried, filtered, concentrated and purified by dry column chromatography to give 2 g of product (IV-B), N-[1-(2-methylindolino)]-N-[N-[(1S)-1-ethoxy-carbonyl-3-phenylpropyl]-N-(2,2,2-trichloroethoxycarbonyl)-L-alanyl]glycine t-butyl ester:

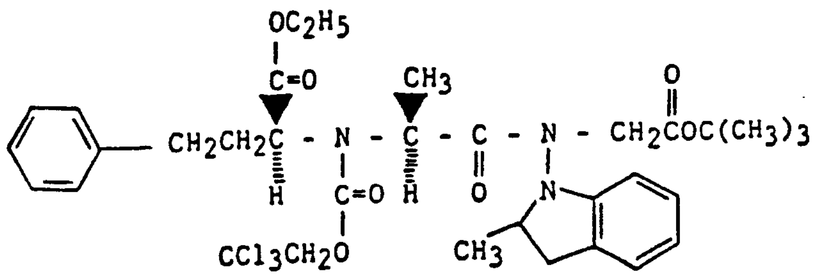

(IV-B)

To a solution of 2 g of product (IV-B) in 10 ml of acid was added 4 g of zinc dust. After 2 h the reaction mixture was filtered through celite and concentrated in vacuo. The residue was purified by dry column chromatography to give 0.8 g of oily product (IV-C), N-[1-(2-methylindolino)]-N-[N-[(1S)-1-ethoxycarbonyl-3-phenylpropyl]-L-alanyl]glycine t-butyl ester:

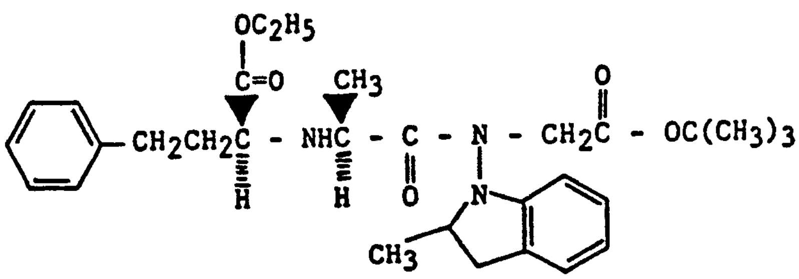

(IV-C)

The dihydrochloric acid salt of the free acid was prepared by bubbling dry hydrogen chloride gas into an etheral solution containing 0.8 g of product (IV-C) at 0°C for 2 h. Concentration in vacuo gave 0.4 g of solid product.

Example IV

The following compounds are prepared by procedures wholly analogous to the procedures used in Example II:

N-(1-piperidino)-N-[N-[(1S)-1-ethoxycarbonyl-3-phenyl-propyl]-L-alanyl]glycine t-butyl ester and dihydrochloric acid salt.

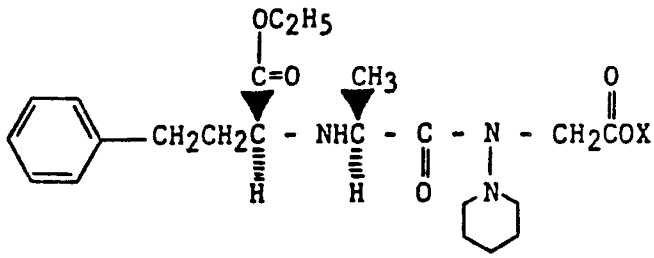

(V-A) X: $-C(CH_3)_3$

(V-B) X: $- H\cdot 2HCl$

Example V

The following compounds are made by procedures wholly analogous to the procedure set forth in Examples I:

(VI-A) N-(N-Ethylbenzylamino)-N-[N-[(1S)-1-ethoxy-carbonyl-3-phentylpropyl]-L-alanyl]glycine:

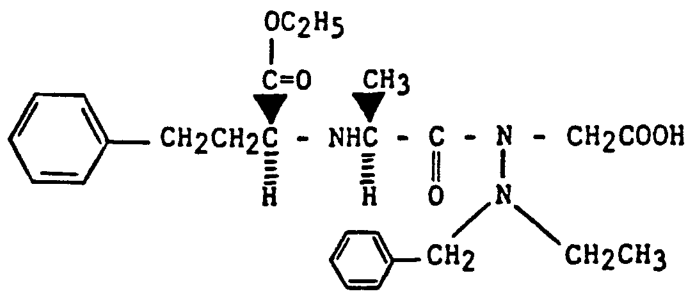

(VI-A)

(VII-A) N-(N-Methylanilino)-N-[N-[(1S)-1-ethoxy-carbonyl-3-phenylpropyl]-L-lysyl]glycine:

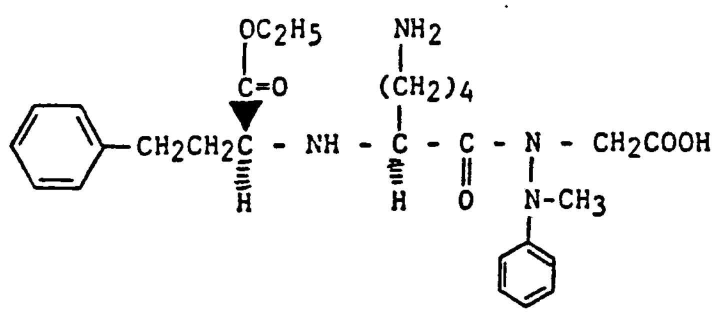

(VII-A)

(VIII-A) N-(N-Methylanilino)-N-[N-[(1S)-1-ethoxy-carbonyl-ethyl]-L-alanyl]glycine

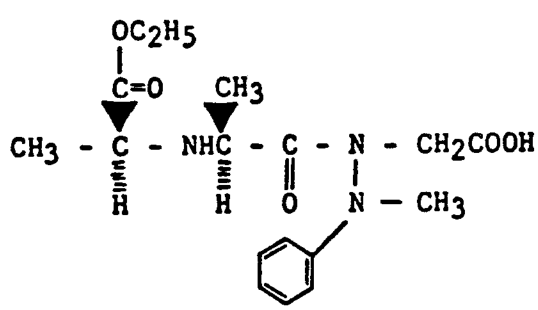

(VIII-A)

(IX-A) N-(Dimethylamino)-N-[N-[(1S)-1-ethoxycarbonyl-3-phenyl-propyl]-L-alanyl]glycine

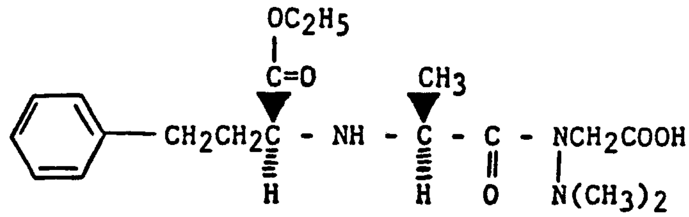

(IX-A)

(X-A) N-(N-methyl-N-(4-pyridine)amino)-N-[N-(1S)-1-ethoxy-carbonyl-3-phenylpropyl]-L-alanyl]glycine

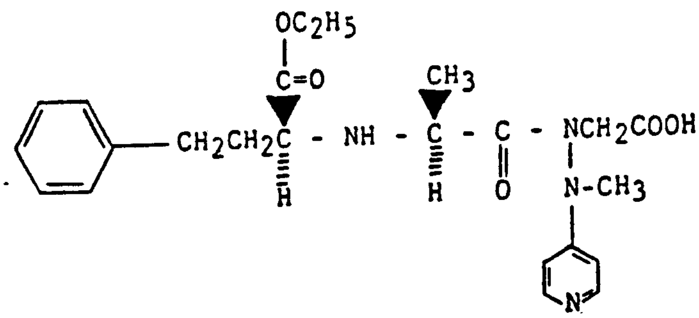

(X-A)

(XI-A) N-(N-phenylamino)-N-(N-(1S)-1-ethoxycarbonyl-3-phenylpropyl]-L-alanyl]glycine

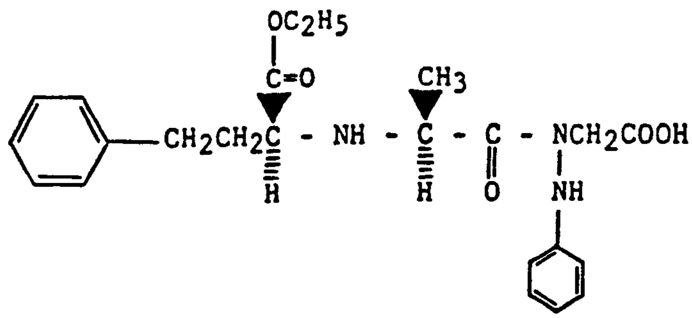

(XI-A)

(XII-A) N-(N-methylanilino)-N-[N-(1S)-1-ethoxycarbonyl-2-phenoxyethyl]-L-alanyl]glycine

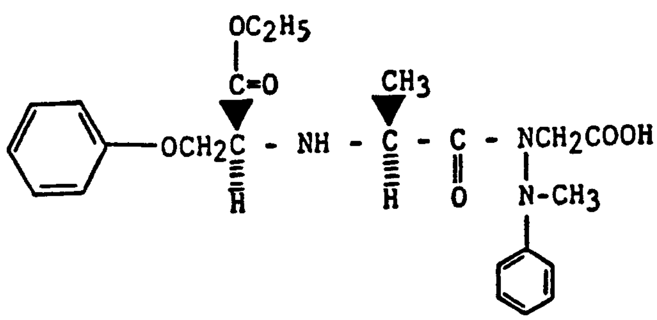

(XII-A)

Example VI

N-(morpholin-1-yl)-N-[N-[(1S)-1-ethoxycarbonyl-3-phenylpropyl]-L-alanyl]glycine dihydrochloride

To a mixture of N-[(1S)-ethoxycarbonyl-3-phenylpropyl]-L-alanine (10 g) and sulfuric acid (10 ml) in 100 ml of dioxane was added 150 ml of isobutylene, and the resulting reaction mixture was shaken in a pressure bottle overnight. The reaction mixture was neutralized with 50% NaOH, taken up in 200 ml of ethyl acetate, and washed with water. The organic solution was dried and evaporated to dryness to give 10 g of an oily product (13-A), N-[(1S)-Ethoxycarbonyl-3-phenylpropyl]-L-alanine t-butyl ester.

A mixture containing 2.52 g (7.51 mmol) of compound (13-A), 1.10 ml (7.99 mmol) of 2,2,2-trichloroethyl chloroformate, and 1.0 ml (12.4 mmol) of pyridine in 25 ml of dry tetrahydrofuran was refluxed under a nitrogen atmosphere for 3 h.

The reaction mixture was filtered, taken up in 200 ml of ether, and washed four times with 1N hydrochloric acid and once with brine. The organic phase was dried over $MgSO_4$, filtered, and concentrated in

vacuo to yield 3.79 g (99%) of compound (13-B), N-[(1S)-1-ethoxycarbonyl-3-phenylpropyl]-N-(2,2,2-trichloroethoxycarbonyl)-L-alanine t-butyl ester.

A mixture containing 1.98 g (3.88 mmol) of compound (13-B) in 25 ml of 4N HCl in dioxane at room temperature and under a nitrogen atmosphere was stirred for 8 h. The mixture was then concentrated in vacuo to provide 1.77 g (100%) of compound (13-C), N-[(1S)-1-ethoxycarbonyl-3-phenylpropyl]-N-(2,2,2-trichloroethoxycarbonyl)-L-alanine.

To a mixture containing 908 mg (2.00 mmol) of compound (13-C) and 0.40 ml (4.6 mmol) of oxalyl chloride in 10 ml of dry methylene chloride at room temperature and under a nitrogen atmosphere was added 10 ml (0.13 mmol) of N,N-dimethylformamide. After 2 h the mixture was carefully concentrated in vacuo (T<30°C) to give 900 ml of compound (13-D), N-[(1S)-ethoxycarbonyl-3-phenylpropyl]-N-(2,2,2-trichloroethoxycarbonyl)-L-alanyl acid chloride.

A mixture of N-aminomorpholine (4 g), t-butyl bromoacetate (7.6 g) and sodium carbonate (2.1 g) in 20 ml of DMF was stirred at room temperature overnight. After concentration in vacuo, the residue was taken up in ethyl acetate. The organic solution was washed with water, dried, filtered and concentrated in vacuo to give 2.5 g of crude N-(morpholin-1-yl)glucine t-butyl ester (compound 13-E). This crude compound was used without further purification. To a solution of 5 g of product (13-D) in 10 ml of methylene chloride was added a solution of 2.5 g of compound (13-E) and 5 ml of pyridine in 5 ml of methylene chloride over a period of 10 min. After 16 h the reaction mixture was washed successively with 1N hydrochloric acid, saturated aqueous sodium bicarbonate, and once with brine. The organic solution was dried, filtered, concentrated in vacuo, and purified by dry column chromatography to give 3.5 g of oily product (13-F), N-(morpholin-1-yl)-N-[N-[(1S)-1-ethoxycarbonyl-3-phenylpropyl]-N-(2,2,2-trichloroethoxycarbonyl)-L-alanyl]glycine t-butyl ester.

To a solution of 0.6 g of product (13-F) in 10 ml of glacial acetic acid was added 2 g of zinc dust. After 3 h the mixture was filtered through celite and concentrated in vacuo. The residue was taken up in ethyl acetate and washed with water. The organic solution was dried, filtered, concentrated in vacuo, and chromatographed to give 0.4 g of product (13-G), N-(morpholin-1-yl)-N-[N-[(1S)-1-ethoxycarbonyl-3-phenylpropyl]L-alanyl]glycine t-butyl ester.

The dihydrochloric acid salt of the free acid was formed by bubbling dry hydrogen chloride gas into an etheral solution containing 0.5 g of product (13-G) at 0°C for 2.5 h.

Concentration in vacuo gave 0.4 g of solid product, m.p. 110°C (decomposes).

## Example VII

N-[Nα-[(1S)-ethoxycarbonyl-3-phenylpropyl]-L-lysyl]-N-(morpholin-1-yl)glycine dihydrobromide

To a mixture of Nα-[(1S)-ethoxycarbonyl-3-phenylpropyl]-Nα(-(2,2,2-trichloroethoxycarbonyl)-Nε-carbobenzyloxy-L-lysine (3.95 g) (prepared by a procedure analogous to compound (13-C)) and 1.2 ml of oxalyl chloride in 25 ml of methylene chloride was added a few drops of dimethyl formamide in 1 ml of methylene chloride under a nitrogen atmosphere. After stirring the mixture for 2 h the solvent was removed and the residue dissolved in 30 ml of methylene chloride. This mixture was added dropwise to a solution of 2 g of compound (13-E) and 1.8 ml of pyridine in 10 ml of methylene chloride. The resulting mixture was stirred for 18 h. The reaction mixture was taken up in 200 ml of ether and washed successively in 30 ml portions of saturated aqueous sodium bicarbonate, 1N HCl, saturated aqueous sodium bicarbonate, and 50% brine. Removal of solvent after drying yielded 5.7 g of crude product which was purified by dry column chromatography (hexane: ethyl acetate = 2:1) to yield 1.5 g of N-[Nα[(1S)-ethoxycarbonyl-3-phenylpropyl]-Nα-(2,2,2-trichloroethoxycarbonyl)-Nεcarbobenzyloxy-L-lysyl]-N-(morpholin-1-yl)glycine t-butyl ester. This product was mixed in 15 ml of glacial acetic acid to which 1.8 g of zinc dust was added, to remove the Nα-protecting group. This mixture was stirred overnight at room temperature. Removal of solvent in vacuo yielded 1.3 g of the crude mixture as a pale yellow oil. The crude mixture was purified by dry column chromatography (ethyl acetate: hexanes = 2.1) to provide 450 mg of N-[Nα-[(1S)-ethoxycarbonyl-3-phenylpropyl]-Nε-carbobenzyloxy-L-lysyl]-N-morpholin-1-yl)glycine t-butyl ester. The Nε-protecting group was removed, and the ester was converted to the free acid hydrobromide, by adding 1.5 ml of a 50:50 mixture of HBr in acetic acid to 450 mg of the product from the preceding step, and letting the mixture stand for 1 h at room temperature. To this mixture anhydrous ether was then added dropwise with swirling. A white precipitate formed which was collected by filtration. The precipitate was washed with ether and dried under vacuum. The product was 350 mg of N-[Nα-[(1S)-ethoxycarbonyl-3-phenylpropyl]-L-lysyl]-N- (morpholin-1-yl)glycine dihydrobromide, m.p. 165°C (decomposes).

## Example VIII

N-[1-(2-methylindolin-1-yl)]-N-[N-[(1S)-ethoxycarbonyl-3-phenylpropyl]-L-alanyl]glycine dihydrochloride

A mixture of 1-amino-2-methylindoline (3.7 g), potassium carbonate (2.8 g) and t-butyl bromoacetate (4 g) in 10 ml of DMF was stirred at room temperature overnight. After removal of DMF in vacuo, the residue was taken up in ethyl acetate. The organic solution was washed with water, dried with magnesium sulfate, filtered, and concentrated. The product was purified by dry column chromatography to give 1.5 g of slightly impure oily product (14-A), N-[1-(2-methylindolin-1-yl]glycine t-butyl ester. This was used for the next reaction without further purification.

A solution of 2.25 g of acid chloride product (13-D) in 10 ml of methylene chloride was added dropwise to a 15 ml of methylene chloride solution containing 1.5 g of product (14-A) and 0.4 g of pyridine over a period of 10 min. After 20 h the reaction mixture was washed successively with 1N hydrochloric acid, saturated aqueous sodium bicarbonate, and brine solution. The organic solution was dried, filtered, concentrated and purified by dry column chromatography to give 2 g of product (14-B), N-[1-(2-methyl-indolin-1-yl]-N-[N-[(1S)-1-ethoxy-carbonyl-3-phenylpropyl]-N-(2,2,2-trichloroethoxycarbonyl)-L-alanyl]-glycine t-butyl ester.

To a solution of 2 g of the product (14-B) in 10 ml of acetic acid was added 4 g of zinc dust. After 2 h the reaction mixture was filtered through celite and concentrated in vacuo. The residue was purified by dry column chromatography to give 0.8 g of oily product (14-C), N-[1-(2-methylindolin-1-yl)]-N-[N-[(1S)-1-ethoxycarbonyl-3-phenylpropyl]-L-alanyl]glycine t-butyl ester:

The dihydrochloric acid salt of the free acid was prepared by bubbling dry hydrogen chloride gas into an etheral solution containing 0.8 g of product (14-C) at 0°C for 2 h.

Concentration in vacuo gave 0.4 g of solid product, m.p. 60—64°C.

## Example IX

N-[N-[(1S)-Ethoxycarbonyl-3-phenylpropyl]-L-alanyl]-N-(pyrrol-1-yl)glycine hydrochloride

Using the procedure employed in Example XIII compound (13-C) (5 g) was reacted with 0.96 ml of oxalyl chloride to form the acid chloride, which was reacted with N-(pyrrol-1-yl) glycine t-butyl ester which had previously been made by the procedure employed to make compound (13-E) using N-aminopyrrole instead of N-aminomorpholine. The reaction product (4 g) was N-protected in 20 ml of glacial acetic acid with 3.1 g of zinc dust, and this mixture was stirred at room temperature overnight under a nitrogen atmosphere. After removal of solvent and washing, which yielded 2.2 g of a reddish-brown oil, this product was purified by dry column chromatography (25% ethyl acetate in hexane) to yield 0.7 g of a pale yellow oil, N-[N-[(1S)-ethoxycarbonyl-3-phenylpropyl]-L-alanyl]-N-(pyrrol-1-yl)glycine t-butyl ester. This product was dissolved in 25 ml of ether in an ice bath, HCl gas was bubbled through the solution for 3 h, and the solution was then allowed to stand for 1.5 h more. Removal of solvent gave 390 mg of a slightly orange powder, N-[N-[(1S)-ethoxycarbonyl-3-phenylpropyl]-L-alanyl]-(pyrrol-1-yl)glycine hydrochloride, m.p. 110—118°C (decomposes).

## Example X

N-(4-Methylpiperazin-1-yl)-N-[N-[(1S)-ethoxycarbonyl-3-phenylpropyl]-L-alanyl]glycine t-butyl ester and the free acid are made by the same procedure employed for Example VI substituting N-amino-N'-methylpiperazine for N- aminomorpholine.

## Example XI

N-(Tetrahydro-1,4-thiazin-4-yl)-N-[N-[(1S)-1-ethoxycarbonyl-3-phenylpropyl]-L-alanyl]glycine t-butyl ester and the free acid are made by the same procedure employed for Example VI substituting N-amino-tetrahydro-1,4-thiazine for N-aminomorpholine.

## Example XII

N-(Thiazolidin-3-yl)-N-[N-[N(1S]-1-ethoxycarbonyl-3-phenylpropyl]-L-alanyl]glycine t-butyl ester and the free acid are made by the same procedure employed for Example VI substituting N-amino-thiazolidine for N-aminomorpholine.

## Example XIII

N-(Piperidin-1-yl)N-[N-[(1S)-1-ethoxycarbonyl-3-phenylpropyl]-L-alanyl]glycine t-butyl ester and the free acid are made by the same procedure employed for Example VI, substituting N-amino-piperidine for N-aminomorpholine.

## Example XIV

N-(Pyrrolidin-1-yl)-N-[N-[(1S)-1-ethoxycarbonyl-3--phenylpropyl]-L-alanyl]glycine t-butyl ester and the free acid are made by the same procedure employed for Example VI substituting N-amino-pyrrolidine for N-aminomorpholine.

## Example XV

N-(2,3-Dihydroindol-1-yl)N-[N-[(1S)-ethoxycarbonyl-3-phenylpropyl]-L-alanyl]glycine t-butyl ester and the free acid are made by the same procedure employed for Example VI, substituting N-amino-2,3-dihydro-indole for N-aminomorpholine.

## Example XVI

N-(1,2,3,4-Tetrahydroisoquinolin-2-yl)-N-[N-[(1S)-1-ethoxycarbonyl-3-phenylpropyl]-L-alanyl]glycine t-butyl ester and the free acid are made by the same procedure employed for Example VI substituting N-amino-1,2,3,4-tetrahydroisoquinoline for N-aminomorpholine.

Example XVII

N-(Imidazol-1-yl)-N-[N-[(1S)-1-ethoxycarbonyl-3-phenylpropyl]-L-alanyl]glycine t-butyl ester and the free acid are made by the same procedure employed for Example VI, substituting N-amino-imidazole for N-aminomorpholine.

**Claims for the Contracting States: BE CH DE FR GB IT LI LU NL SE**

1. Compounds of the general formula I

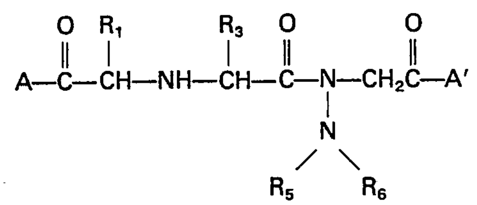

(I)

and their pharmaceutically acceptable salts wherein

A and A' are independently hydroxy or $C_1$—$C_6$-alkoxy;

$R_1$ is $C_1$—$C_9$-alkyl, arylalkyl or aryloxyalkyl and 1 to 9 carbon atoms in the alkyl moiety; and

$R_3$ is $C_1$—$C_6$-alkyl, or ω-amino $C_1$—$C_6$-alkyl,

$R_5$ and $R_6$ are independently hydrogen, $C_1$—$C_9$-alkyl aryl, aralkyl with 1 to 9 carbon atoms in the alkyl moiety or pyridyl, or

$R_5$ and $R_6$ when taken together with the nitrogen atom to which they are attached form a substituted or unsubstituted, saturated or unsaturated monocyclic or unsubstituted, saturated or unsaturated monocyclic or polycyclic ring containing from 2 to 9 carbon atoms which may include one oxygen, sulfur or additional nitrogen atom.

2. The compound of Claim 1 which is one of the following:

N-(N-methylanilino)-N-[N-[(1S)-1-ethoxycarbonyl-3-phenylpropyl]-L-alanyl]-glycine t-butyl ester.

N-(N-methylanilino)-N-[N-[(1S)-1-ethoxycarbonyl-3-phenylpropyl]-L-alanyl]-glycine dihydrochloric acid salt.

N-(morpholin-1-yl)-N-[N-[(1S)-1-ethoxycarbonyl-3-phenylpropyl]-L-alanyl]glycine t-butyl ester.

N-[Nα-[(1S)-ethoxycarbonyl-3-phenylpropyl]-L-lysyl]-N-[morpholin-1-yl)glycine dihydrobromide.

N-[1-(2-methyl-indolin-1-yl)]-N-[N-[(1S)-1-ethoxycarbonyl-3-phenylpropyl]-L-alanyl]glycine dihydrochloride.

N-[N-[(1S)-ethoxycarbonyl-3-phenylpropyl]-L-alanyl]-N-(pyrrol-1-yl)glycine hydrochloride.

N-(4-methyl-piperazin-1-yl)-N-[N-[(1S)-1-ethoxycarbonyl-3-phenylpropyl]-L-alanyl]glycine t-butyl ester.

N-(tetrahydro-1,4-thiazin-4-yl)-N-[N-[(1S)-1-ethoxycarbonyl-3-phenylpropyl]-L-alanyl]glycine t-butyl ester.

N-(thiazolidin-3-yl)-N-[(1S)-1-ethoxycarbonyl-3-phenylpropyl]-L-alanyl]glycine t-butyl ester.

N-(piperidin-1-yl)-N-[N-[N-[(1S)-1-ethoxycarbonyl-3-phenylpropyl]-L-alanyl]-glycine t-butyl ester.

N-(pyrrolidin-1-yl)-N-[N-[(1S)-1-ethoxycarbonyl-3-phenylpropyl]-L-alanyl]-glycine t-butyl ester.

N-(2,3-dihydroindol-1-yl)-N-[N-[(1S)-1-ethoxycarbonyl-3-phenylpropyl]-L-alanyl]glycine t-butyl ester.

N-(1,2,3,4-tetrahydroisoquinolin-2-yl)-N-[N-[(1S)-1-ethoxycarbonyl-3-phenylpropyl]-L-alanyl]glycine t-butyl ester.

N-(imidazol-1-yl)-N-[N-(1S)-1-ethoxycarbonyl-3-phenylpropyl[-L-alanyl]glycine t-butyl ester.

N-(morpholine-1-yl)-N-[N-(1S)-1-ethoxycarbonyl-3-phenylpropyl]-L-alanyl]glycine dihydrochloric acid salt.

N-[1-(2-methylindolin-1-yl)]-N-[(1S)-1-ethoxycarbonyl-3-phenylpropyl]-L-alanyl]glycine t-butyl ester.

N-[1-(2-methylindolin-1-yl)]-N-[N-[(1S)-1-ethoxycarbonyl-3-phenylpropyl]-L-alanyl]glycine dihydrochloric acid salt.

N-(1-piperidino)-N-[N-[(1S)-1-ethoxycarbonyl-3-phenylpropyl]-L-alanyl]glycine t-butyl ester.

N-(1-piperidino)-N-[N-[(1S)-1-ethoxycarbonyl-3-phenylpropyl]-L-alanyl]glycine dihydrochloric acid salt.

N-(N-ethylbenzylamino-N-[N-(1S)-1-ethoxycarbonyl-3-phenyl-propyl]-L-alanyl]glycine.

N-(N-methylanilino)-N-[N-[(1S)-1-ethoxycarbonyl-3-phenylpropyl]-L-lysinyl]-glycine.

N-(N-methylanilino)-N-[N-[(1S)-1-ethoxycarbonylethyl]-L-alanyl]glycine.

N-(dimethylamino)-N-[N-[(1S)-1-ethoxycarbonyl-3-phenylpropyl]-L-alanyl]glycine.

N-(N-methyl-N-(4-pyridine)amino)-N-[N-(1S)-1-ethoxycarbonyl-3-phenylpropyl]-L-alanyl]glycine.

N-(N-phenylamino)-N-[N-(1S)-1-ethoxycarbonyl-3-phenylpropyl]-L-alanyl]-glycine.

N-(N-methylanilino)-N-[N-(1S)-1-ethoxycarbonyl-2-phenoxyethyl]-L-alanyl]-glycine.

N-(morpholin-1-yl)-N-[N-[(1S)-1-ethoxycarbonyl-3-phenylpropyl]-L-alanyl]-glycine dihydrochloride.

3. A pharmaceutical preparation comprising at least one compound or salt according to claim 1 or 2 in association with a pharmaceutically acceptable carrier.

4. A process for the preparation of compounds of claim 1 or 2 which comprises reacting under amide-forming conditions an amino compound of formula II

$$R_5R_6N—NH—CH_2—COR_9 \qquad II$$

wherein $R_5$ and $R_6$ are as defined in claim 1 and $R_9$ is hydrogen or lower alkyl, with an acylating derivative of an acid of formula III

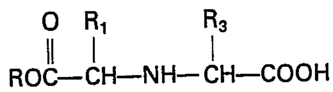

III

wherein $R_1$ and $R_3$ are as defined in claim 1 and R is hydrogen or lower alkyl, or reacting a dipeptide of formula IV

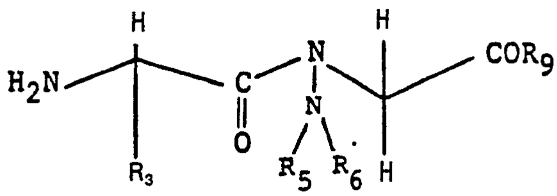

IV

wherein $R_3$, $R_5$ and $R_6$ are as defined in claim 1 and $R_9$ is as described above, with an α-keto-acid or ester of formula V

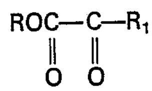

V

wherein $R_1$ is as defined in claim 1 and R is as defined above, to form the corresponding imine and reducing said amine, or reacting a compound of formula VI

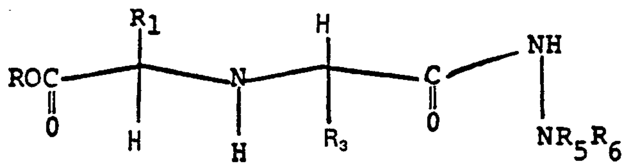

VI

wherein $R_1$, $R_3$, $R_5$ and $R_6$ are as defined as in claim 1 and R is as defined above with an α-halo compound of formula VII

$$Hal—CH_2—COR_9 \qquad VII$$

wherein $R_9$ is as defined above and Hal is halogen, and optionally by substitution or conversion reactions introducing various substituents into the said products; and optionally forming salts thereof, especially pharmaceutically acceptable salts with acids and bases.

**Claims for the Contracting State: AT**

1. A process for the preparation of compounds of the general formula I

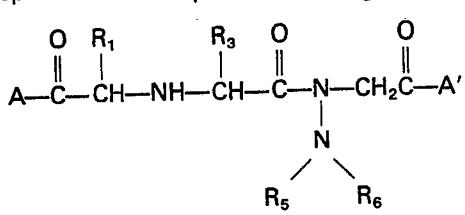

(I)

and their pharmaceutically acceptable salts wherein
A and A' are independently hydroxy or $C_1$—$C_6$-alkoxy;
$R_1$ is $C_1$—$C_9$-alkyl, arylalkyl or aryloxyalkyl with 1 to 9 carbon atoms in the alkyl moiety; and
$R_3$ is $C_1$—$C_6$-alkyl, or ω-amino $C_1$—$C_6$-alkyl,
$R_5$ and $R_6$ are independently hydrogen, $C_1$—$C_9$-alkyl aryl, aralkyl with 1 to 9 carbon atoms in the alkyl moiety or pyridyl, or

$R_5$ and $R_6$ when taken together with the nitrogen atom to which they are attached form a substituted or unsubstituted, saturated or unsaturated monocyclic or polycyclic ring containing from 2 to 9 carbon atoms which may include one oxygen, sulfur or additional nitrogen atom.
which comprises reacting under amide-forming conditions an amino compound of formula II

$$R_5R_6N—NH—CH_2—COR_9 \qquad II$$

wherein $R_5$ and $R_6$ are as defined in claim 1 and $R_9$ is hydrogen or lower alkyl, with an acylating derivative of an acid of formula III

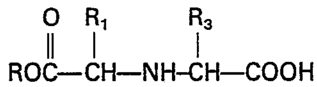

III

wherein $R_1$ and $R_3$ are as defined in claim 1 and R is hydrogen or lower alkyl, or reacting a depeptide of formula IV

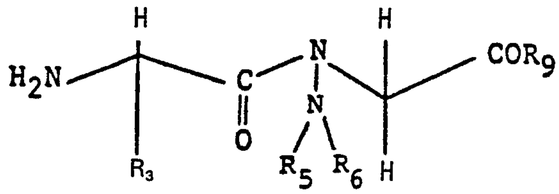

IV

wherein $R_3$, $R_5$ and $R_6$ are as defined in claim 1 and $R_9$ is as described above, with an α-keto-acid or ester of formula V

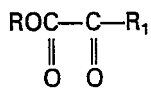

V

wherein $R_1$ is as defined in claim 1 and R is as defined above, to form the corresponding imine and reducing said amine, or reacting a compound of formula VI

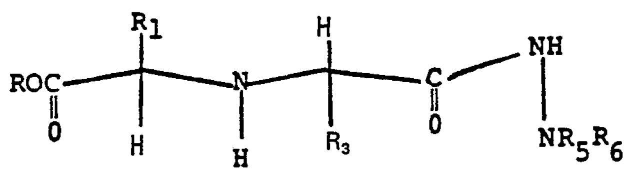

VI

wherein $R_1$, $R_3$, $R_5$ and $R_6$ are as defined as in claim 1 and R is as defined above with an α-halo compound of formula VII

$$Hal—CH_2—COR_9 \qquad VII$$

wherein $R_9$ is as defined above and Hal is halogen, and optionally by substitution or conversion reactions introducing various substituents into the said products; and optionally forming salts thereof, especially pharmaceutically acceptable salts with acids and bases.

2. The process of claim 1 characterised in that one of the following compounds is prepared:

N-(N-methylanilino)-N-[N-[(1S)-1-ethoxycarbonyl-3-phenylpropyl]-L-alanyl]-glycine t-butyl ester.

N-(N-methylanilino)-N-[N-[(1S)-1-ethoxycarbonyl-3-phenylpropyl]-L-alanyl]-glycine dihydrochloric acid salt.

N-(morpholin-1-yl)-N-[N-[(1S)-1-ethoxycarbonyl-3-phenylpropyl]-L-alanyl]glycine t-butyl ester.

N-[Nα-[(1S)-ethoxycarbonyl-3-phenylpropyl]-L-lysyl]-N-[morpholin-1-yl)glycine dihydrobromide.

N-[1-(2-methyl-indolin-1-yl)-N-[N-[(1S)-1-ethoxycarbonyl-3-phenylpropyl]-L-alanyl]glycine dihydrochloride.

N-[N-[(1S)-ethoxycarbonyl-3-phenylpropyl]-L-alanyl]-N-(pyrrol-1-yl)glycine hydrochloride.

N-(4-methyl-piperazin-1-yl)-N-[N-[(1S)-1-ethoxycarbonyl-3-phenylpropyl]-L-alanyl]glycine t-butyl ester.

N-(tetrahydro-1,4-thiazin-4-yl)-N-[N-[(1S)-1-ethoxycarbonyl-3-phenylpropyl]-L-alanyl]glycine t-butyl ester.

N-(thiazolidin-3-yl)-N-[(1S)-1-ethoxycarbonyl-3-phenylpropyl]-L-alanyl]glycine t-butyl ester.

N-(piperidin-1-yl)-N-[N-[N-[(1S)-1-ethoxycarbonyl-3-phenylpropyl]-L-alanyl]-glycine t-butyl ester.
N-(pyrrolidin-1-yl)-N-[N-[(1S)-1-ethoxycarbonyl-3-phenylpropyl]-L-alanyl]-glycine t-butyl ester.
N-(2,3-dihydroindol-1-yl)-N-[N-[(1S)-1-ethoxycarbonyl-3-phenylpropyl]-L-alanyl]glycine t-butyl ester.
N-(1,2,3,4-tetrahydroisoquinolin-2-yl)-N-[N-[(1S)-1-ethoxycarbonyl-3-phenylpropyl]-L-alanyl]glycine t-butyl ester.
N-(imidazol-1-yl)-N-[N-(1S)-1-ethoxycarbonyl-3-phenylpropyl[-L-alanyl]glycine t-butyl ester.
N-(morpholine-1-yl)-N-[N-(1S)-1-ethoxycarbonyl-3-phenylpropyl]-L-alanyl]glycine dihydrochloric acid salt.
N-[1-(2-methylindolin-1-yl]-N-[N-(1S)-1-ethoxycarbonyl-3-phenylpropyl]-L-alanyl]glycine t-butyl ester.
N-[1-(2-methylindolin-1-yl)]-N-[N-[(1S)-1-ethoxycarbonyl-3-phenylpropyl]-L-alanyl]glycine dihydrochloric acid salt.
N-(1-piperidino)-N-[N-[(1S)-1-ethoxycarbonyl-3-phenylpropyl]-L-alanyl]glycine t-butyl ester.
N-(1-piperidino)-N-[N-[(1S)-1-ethoxycarbonyl-3-phenylpropyl]-L-alanyl]glycine dihydrochloric acid salt.
N-(N-ethylbenzylamino-N-[N-[(1S)-1-ethoxycarbonyl-3-phenyl-propyl]-L-alanyl]glycine.
N-(N-methylanilino)-N-[N-[(1S)-1-ethoxycarbonyl-3-phenylpropyl]-L-lysinyl]-glycine.
N-(N-methylanilino)-N-[N-[(1S)-1-ethoxycarbonylethyl]-L-alanyl]glycine.
N-(dimethylamino)-N-[N-[(1S)-1-ethoxycarbonyl-3-phenylpropyl]-L-alanyl]glycine.
N-(N-methyl-N-(4-pyridine)amino)-N-[N-(1S)-1-ethoxycarbonyl-3-phenylpropyl]-L-alanyl]glycine.
N-(N-phenylamino)-N-[N-(1S)-1-ethoxycarbonyl-3-phenylpropyl]-L-alanyl]-glycine.
N-(N-methylanilino)-N-[N-(1S)-1-ethoxycarbonyl-2-phenoxyethyl]-L-alanyl]-glycine.
N-(morpholin-1-yl)-N-[N-[(1S)-1-ethoxycarbonyl-3-phenylpropyl]-L-alanyl]-glycine dihydrochloride.

3. A process for the preparation of a pharmaceutical preparation comprising at least one compound or salt according to claim 1 or 2 as active ingredient comprising formulating the active ingredient with at least one pharmaceutically acceptable carrier and/or at least one usual diluent.

**Patentansprüche für die Vertragsstaaten: BE CH DE FR GB IT LI LU NL SE**

1. Verbindungen der allgemeinen Formel I

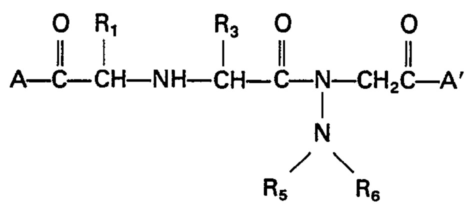

(I)

und ihre pharmazeutisch annehmbaren Salze, worin

A und A' unabhängig voneinander Hydroxy oder $C_1$—$C_6$-Alkoxy sind,

$R_1$ $C_1$—$C_9$-Alkyl, Arylalkyl oder Aryloxyalkyl mit 1 bis 9 Kohlenstoffatomen in dem Alkylrest ist und $R_3$ $C_1$—$C_6$-Alkyl oder ω-Amino-$C_1$—$C_6$-alkyl ist,

$R_5$ und $R_6$ unabhängig voneinander Wasserstoff, $C_1$—$C_9$-Alkyl, Aryl, Aralkyl mit 1 bis 9 Kohlenstoffatomen in dem Alkylrest oder Pyridyl sind oder

$R_5$ und $R_6$ zusammen mit dem Stickstoffatom, an das sie gebunden sind, einen substituierten oder unsubstituierten, gesätiggten oder ungesättigten, monocyclischen oder polycyclischen Ring, enthaltend 2 bis 9 Kohlenstoffatome, bilden, welcher ein Sauerstoff-, Schwefel- oder weiteres Stickstoffatom einschließen kann.

2. Verbindung nach Anspruch 1, welche eine der folgenden ist:
N-(N-Methylanilino)-N-[N-[(1S]-1-ethoxycarbonyl-3-phenylpropyl]-L-alanyl]-glycin-t-butylester.
N-(N-Methylanilino)-N-[N-[(1S)-1-ethoxycarbonyl-3-phenylpropyl]-L-alanyl]-glycin-disalzsäuresalz.
N-(Morpholin-1-yl)-N-[N-[(1S)-1-ethoxycarbonyl-3-phenylpropyl]-L-alanyl]glycin-t-butylester.
N-[Nα-[(1S)-Ethoxycarbonyl-3-phenylpropyl]-L-lysyl]-N-morpholin-1-yl)glycin-dihydrobromid.
N-[1-(2-Methylindolin-1-yl)]-N-[N-[(1S)-1-ethoxycarbonyl-3-phenylpropyl]-L-alanyl]glycin-dihydrochlorid.
N-[N-(1S)-Ethoxycarbonyl-3-phenylpropyl]-L-alanyl]-N-(pyrrol-1-yl)glycin-hydrochlorid.
N-(4-Methylpiperazin-1-yl)-N-[N-[(1S)-1-ethoxycarbonyl-3-phenylpropyl]-L-alanyl]glycin-t-butylester.
N-(Tetrahydro-1,4-thiazin-4-yl)-N-[N-[(1S]-1-ethoxycarbonyl-3-phenylpropyl]-L-alanyl]glycin-t-butylester.
N-(Thiazolidin-3-yl)-N-[N-[(1S)-1-ethoxycarbonyl-3-phenylpropyl]-L-alanyl]glycin-t-butylester.
N-(Piperidin-1-yl)-N-[N-[N-[(1S)-1-ethoxycarbonyl-3-phenylpropyl]-L-alanyl]-glycin-t-butylester.
N-(Pyrrolidin-1-yl)-N-[N-[(1S)-1-ethoxycarbonyl-3-phenylpropyl]-L-alanyl]-glycin-t-butylester.
N-(2,3-Dihydroindol-1-yl)-N-[N-[(1S)-1-ethoxycarbonyl-3-phenylpropyl]-L-alanyl]glycin-t-butylester.
N-(1,2,3,4-Tetrahydroisochinolin-2-yl)-N-[N-[(1S)-1-ethoxycarbonyl-3-phenylpropyl]-L-alanyl]glycin-t-butylester.

N-(Imidazol-1-yl)-N-[N-[(1S)-1-ethoxycarbonyl-3-phenylpropyl]-[L-alanyl]glycin-t-butylester.
N-(Morpholin-1-yl)-N-[N-(1S)-1-ethoxycarbonyl-3-phenylpropyl]-L-alanyl]glycin-disalzsäuresalz.
N-[1-(2-Methylindolin-1-yl)]-N-[N-[(1S)-1-ethoxycarbonyl-3-phenylpropyl]-L-alanyl]glycin-t-butylester.
N-[1-(2-Methylindolin-1-yl)]-N-[N-[(1S)-1-ethoxycarbonyl-3-phenylpropyl]-L-alanyl]glycin-disalzsäuresalz.
N-(1-Piperidino)-N-[N-[(1S)-1-ethoxycarbonyl-3-phenylpropyl]-L-alanylglycin]-t-butylester.
N-(1-Piperidino)-N-[N-[(1S)-1-ethoxycarbonyl-3-phenylpropyl]-L-alanyl]glycin-disalzsäuresalz.
N-(N-Ethylbenzylamino-N-[N-[(1S)-1-ethoxycarbonyl-3-phenylpropyl]-L-alanyl]glycin.
N-(N-Methylanilino)-N-[N-[(1S)-1-ethoxycarbonyl-3-phenylpropyl]-L-lysinyl]-glycin.
N-(N-Methylanilino)-N-[N-[(1S)-1-ethoxycarbonylethyl]-L-alanyl]glycin.
N-(Dimethylamino)-N-[N-[(1S)-1-ethoxycarbonyl-3-phenylpropyl]-L-alanyl]glycin.
N-(N-Methyl-N-(4-pyridin)amino)-N-[N-(1S)-1-ethoxycarbonyl-3-phenylpropyl]-L-alanyl]glycin.
N-(N-Phenylamino)-N-[N-(1S)-1-ethoxycarbonyl-3-phenylpropyl]-L-alanyl]glycin.
N-(N-Methylanilino)-N-[N-(1S)-1-ethoxycarbonyl-2-phenoxyethyl]-L-alanyl]glycin.
N-(Morpholin-1-yl)-N-[N-[(1S)-1-ethoxycarbonyl-3-phenylpropyl]-L-alanyl]-glycin-dihydrochlorid.

3. Pharmazeutische Zubereitung, umfassend wenigstens eine Verbindung oder ein Salz nach Anspruch 1 oder 2 in Kombination mit einem pharmazeutisch annehmbaren Träger.

4. Verfahren zur Herstellung der Verbindungen nach Anspruch 1 oder 2, bei dem unter amidbildenden Bedingungen eine Aminoverbindung der Formel II

$$R_5R_6N—NH—CH_2—COR_9 \qquad II$$

worin $R_5$ und $R_6$ wie in Anspruch 1 definiert sind und $R_9$ Wasserstoff oder Niedrigalkyl ist, mit einem Acylierungsderivat einer Säure der Formel III

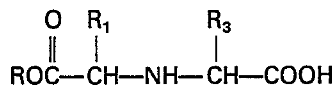
III

worin $R_1$ und $R_3$ wie in Anspruch 1 definiert sind und R Wasserstoff oder Niedrigalkyl ist, umgesetzt wird oder
ein Dipeptid der Formel IV

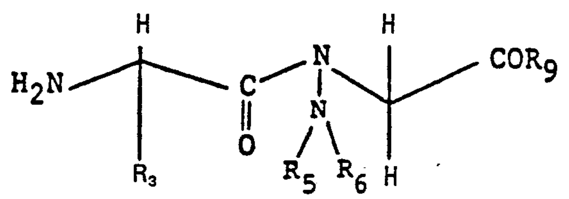
IV

worin $R_3$, $R_5$ und $R_6$ wie in Anspruch 1 definiert sind und $R_9$ wie vorstehend definiert ist,
mit einer α-Ketosäure oder einem Ester der Formel V

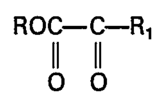
V

worin $R_1$ wie in Anspruch 1 definiert ist und R wie vorstehend definiert ist, umgesetzt wird zur Bildung des entsprechenden Imins und das Imin reduziert wird oder
eine Verbindung der Formel VI

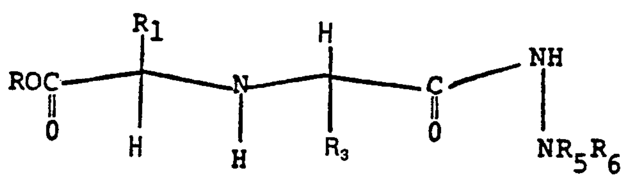
VI

worin $R_1$, $R_3$, $R_5$ und $R_6$ wie in Anspruch 1 definiert sind und R wie vorstehend definiert ist, mit einer α-Halogenverbindung der Formel VII

$$Hal—CH_2—COR_9 \qquad VII$$

worin $R_9$ wie vorstehend definiert ist und Hal Halogen ist, umgesetzt wird und gegebenenfalls durch Substitutions- oder Umwandlungsreaktionen verschiedene Substituenten in die Produkte eingeführt werden und gegebenenfalls Salze davon, insbesondere pharmazeutisch annehmbare Salze, mit Säuren und Basen gebildet werden.

**Patentansprüche für den Vertragsstaat: AT**

1. Verfahren zur Herstellung von Verbindungen der allgemeinen Formel I

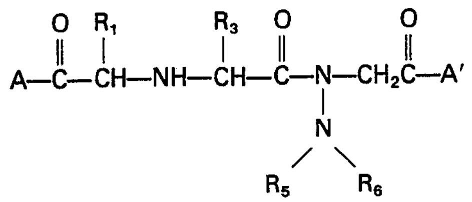

(I)

und ihre pharmazeutisch annehmbaren Salze, worin

A und A' unabhängig voneinander Hydroxy oder $C_1$—$C_6$-Alkoxy sind,

$R_1$ $C_1$—$C_9$-Alkyl, Arylalkyl oder Aryloxyalkyl mit 1 bis 9 Kohlenstoffatomen in dem Alkylrest ist und $R_3$ $C_1$—$C_6$-Alkyl oder ω-Amino-$C_1$—$C_6$-alkyl ist,

$R_5$ und $R_6$ unabhängig voneinander Wasserstoff, $C_1$—$C_9$-Alkyl, Aryl, Aralkyl mit 1 bis 9 Kohlenstoffatomen in dem Alkylrest oder Pyridyl sind oder

$R_5$ und $R_6$ zusammen mit dem Stickstoffatom, an das sie gebunden sind, einen substituierten oder unsubstituierten, gesätiggten oder ungesättigten, monocyclischen oder polycyclischen Ring, enthaltend 2 bis 9 Kohlenstoffatome, bilden, welcher ein Sauerstoff-, Schwefel- oder weiteres Stickstoffatom einschließen kann, bei dem unter amidbildenden Bedingungen eine Aminoverbindung der Formel II

$$R_5R_6N—NH—CH_2—COR_9 \qquad II$$

worin $R_5$ und $R_6$ wie in Anspruch 1 definiert sind und $R_9$ Wasserstoff oder Niedrigalkyl ist, mit einem Acylierungsderivat einer Säure der Formel III

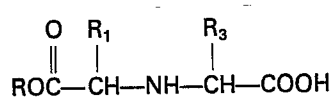

III

worin $R_1$ und $R_3$ wie in Anspruch 1 definiert sind und R Wasserstoff oder Niedrigalkyl ist, umgesetzt wird oder

ein Dipeptid der Formel IV

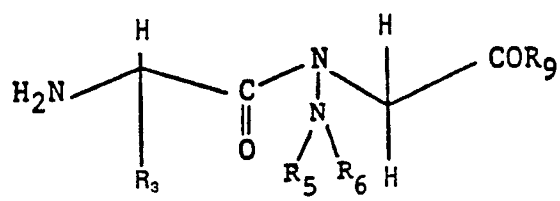

IV

worin $R_3$, $R_5$ und $R_6$ wie in Anspruch 1 definiert sind und $R_9$ wie vorstehend definiert ist, mit einer α-Ketosäure oder einem Ester der Formel V

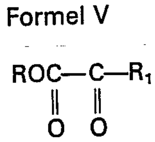

V

worin $R_1$ wie in Anspruch 1 definiert ist und R wie vorstehend definiert ist, umgesetzt wird zur Bildung des entsprechenden Imins und das Imin reduziert wird oder
eine Verbindung der Formel VI

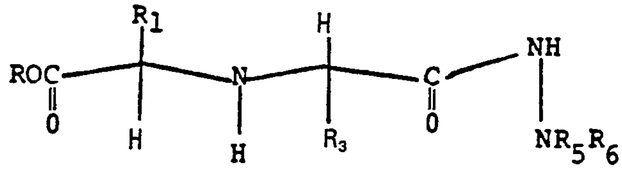

VI

worin $R_1$, $R_3$, $R_5$ und $R_6$ wie in Anspruch 1 definiert sind und R wie vorstehend definiert ist, mit einer α-Halogenverbindung der Formel VII

$$Hal—CH_2—COR_9 \quad VII$$

worin $R_9$ wie vorstehend definiert ist und Hal Halogen ist, umgesetzt wird und gegebenenfalls durch Substitutions- oder Umwandlungsreaktionen verschiedene Substituenten in die Produkte eingeführt werden und gegebenenfalls Salze davon, insbesondere pharmazeutisch annehmbare Salze, mit Säuren und Basen gebildet werden.

2. Verfahren nach Anspruch 1, dadurch gekennzeichnet, daß eine der folgenden Verbindungen hergestellt wird:

N-(N-Methylanilino)-N-[N-[(1S]-1-ethoxycarbonyl-3-phenylpropyl]-L-alanyl]-glycin-t-butylester.
N-(N-Methylanilino)-N-[N-[(1S)-1-ethoxycarbonyl-3-phenylpropyl]-L-alanyl]-glycin-disalzsäuresalz.
N-(Morpholin-1-yl)-N-[N-[(1S)-1-ethoxycarbonyl-3-phenylpropyl]-L-alanyl]glycin-t-butylester.
N-[Nα-[(1S)-Ethoxycarbonyl-3-phenylpropyl]-L-lysyl]-N-morpholin-1-yl)glycin-dihydrobromid.
N-[1-(2-Methylindolin-1-yl)]-N-[N-[(1S)-1-ethoxycarbonyl-3-phenylpropyl]-L-alanyl]glycin-dihydrochlorid.
N-[N-[(1S)-Ethoxycarbonyl-3-phenylpropyl]-L-alanyl]-N-(pyrrol-1-yl)glycinhydrochlorid.
N-(4-Methylpiperazin-1-yl)-N-[N-[(1S)-1-ethoxycarbonyl-3-phenylpropyl]-L-alanyl]glycin-t-butylester.
N-(Tetrahydro-1,4-thiazin-4-yl)-N-[N-[(1S]-1-ethoxycarbonyl-3-phenylpropyl]-L-alanyl]glycin-t-butylester.
N-(Thiazolidin-3-yl)-N-[N-[(1S)-1-ethoxycarbonyl-3-phenylpropyl]-L-alanyl]glycin-t-butylester.
N-(Piperidin-1-yl)-N-[N-[N-[(1S)-1-ethoxycarbonyl-3-phenylpropyl]-L-alanyl]-glycin-t-butylester.
N-(Pyrrolidin-1-yl)-N-[N-[(1S)-1-ethoxycarbonyl-3-phenylpropyl]-L-alanyl]-glycin-t-butylester.
N-(2,3-Dihydroindol-1-yl)-N-[N-[(1S)-1-ethoxycarbonyl-3-phenylpropyl]-L-alanyl]glycin-t-butylester.
N-(1,2,3,4-Tetrahydroisochinolin-2-yl)-N-[N-[(1S)-1-ethoxycarbonyl-3-phenylpropyl]-L-alanyl]glycin-t-butylester.
N-(Imidazol-1-yl)-N-[N-[(1S)-1-ethoxycarbonyl-3-phenylpropyl]-L-alanyl]glycin-t-butylester.
N-(Morpholin-1-yl)-N-[N-[(1S)-1-ethoxycarbonyl-3-phenylpropyl]-L-alanyl]glycin-disalzsäuresalz.
N-[1-(2-Methylindolin-1-yl)]-N-[N-[(1S)-1-ethoxycarbonyl-3-phenylpropyl]-L-alanyl]glycin-t-butylester.
N-[1-(2-Methylindolin-1-yl)]-N-[N-[(1S)-1-ethoxycarbonyl-3-phenylpropyl]-L-alanyl]glycin-disalzsäuresalz.
N-(1-Piperidino)-N-[N-[(1S)-1-ethoxycarbonyl-3-phenylpropyl]-L-alanyl]glycin-t-butylester.
N-(1-Piperidino)-N-[N-[(1S)-1-ethoxycarbonyl-3-phenylpropyl]-L-alanyl]glycin-disalzsäuresalz.
N-(N-Ethylbenzylamino-N-[N-[(1S)-1-ethoxycarbonyl-3-phenylpropyl]-L-alanyl]glycin.
N-(N-Methylanilino)-N-[N-[(1S)-1-ethoxycarbonyl-3-phenylpropyl]-L-lysinyl]-glycin.
N-(N-Methylanilino)-N-[N-[(1S)-1-ethoxycarbonylethyl]-L-alanyl]glycin.
N-(Dimethylamino)-N-[N-[(1S)-1-ethoxycarbonyl-3-phenylpropyl]-L-alanyl]glycin.
N-(N-Methyl-N-(4-pyridin)amino)-N-[N-(1S)-1-ethoxycarbonyl-3-phenylpropyl]-L-alanyl]glycin.
N-(N-Phenylamino)-N-[N-(1S)-1-ethoxycarbonyl-3-phenylpropyl]-L-alanyl]glycin.
N-(N-Methylanilino)-N-[N-(1S)-1-ethoxycarbonyl-2-phenoxyethyl]-L-alanyl]glycin.
N-(Morpholin-1-yl)-N-[N-[(1S)-1-ethoxycarbonyl-3-phenylpropyl]-L-alanyl]-glycin-dihydrochlorid.

3. Verfahren zur Herstellung einer pharmazeutischen Zubereitung, umfassend wenigstens eine Verbindung oder ein Salz nach Anspruch 1 oder 2 als aktiven Bestandteil, bei dem der aktive Bestandteil mit wenigstens einem pharmazeutisch annehmbaren Träger und/oder wenigstens einem üblichen Verdünnungsmittel formuliert wird.

**Revendications pour les Etats contractants: BE CH DE FR GB IT LI LU NL SE**

1. Composés représentés par la formula générale (I):

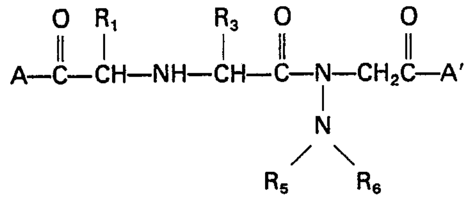

(I)

et leurs sels pharmaceutiquement acceptables, ou:

A et A' représentent indépendamment hydroxy ou alcoxy en $C_1$—$C_6$;

$R_1$ représente alkyle en $C_1$—$C_9$, arylalkyle ou aryloxyalkyle avec 1 à 9 atomes de carbone dans la fraction alkyle; et

$R_3$ représente alkyle en $C_1$—$C_6$, ou ω-amino-alkyle en $C_1$—$C_6$,

$R_5$ et $R_6$ représente indépendamment hydrogène, alkyle en $C_1$—$C_9$, aryle, aralkyle avec 1 à 9 atomes de carbone dans la fraction alkyle, ou pyridyle, ou

$R_5$ et $R_6$, lorsqu'ils sont pris conjointement avec l'atome d'azote auquel ils sont attachés, forment un noyau monocyclique ou polycyclique, saturé ou insaturé, substitué ou non-substitué, contenant de 2 à 9 atomes de carbone, qui peut renfermer un atome d'oxygène, un atome de soufre ou un atome d'azote supplémentaire.

2. Composés selon la revendication 1, qui est l'un des suivants:

Ester tert.-butylique de la N-(N-méthylanilino)-N-[N-[(1S)-éthoxycarbonyl-1 phényl-3 propyl]-L-alanyl]-glycine

Dichlorhydrate de la N-(N-méthylanilino)-N-[N-[(1S)-éthoxycarbonyl-1 phényl-3 propyl]-L-alanyl]-glycine

Ester tert.-butylique de la N-(morpholinyl-1)-N-[N-[(1S)-éthoxycarbonyl-1 phényl-3 propyl]-L-alanyl]-glycine

Dibromhydrate de la N-[Nα-[(1S)-éthoxycarbonyl-1 phényl-3 propyl]-L-lysyl]-N-(morpholinyl-1)glycine.

Dichlorhydrate de la N-[(méthyl-2 indolinyl-1)-1]-N-[N-[(1S)-éthoxycarbonyl-1 phényl-3 propyl]-L-alanyl]glycine

Chlorhydrate de la N-[N-[(1S)-éthoxycarbonyl-1 phényl-3 propyl]-L-alanyl]-N-(pyrrolyl-1)glycine.

Ester tert.-butylique de la N-(méthyl-4 pipérazinyl-1)-N-[N-[(1S)-éthoxycarbonyl-1 phényl-3 propyl]-L-alanyl]glycine

Ester tert.-butylique de la N-(tétrahydrothiazine-1,4-yl-4)-N-[N-[(1S)-éthoxycarbonyl-1 phényl-3 propyl]-L-alanyl]glycine

Ester tert.-butylique de la N-(thiazolidinyl-3)-N-[N-[(1S)-éthoxycarbonyl-1 phényl-3 propyl]-L-alanyl]-glycine

Ester tert.-butylique de la N-(pipéridinyl-1)-N-[N-[N-[(1S)-éthoxycarbonyl-1 phényl-3 propyl]-L-alanyl]-glycine

Ester tert.-butylique de la N-(pyrrolidinyl-1)-N-[N-[(1S)-éthoxycarbonyl-1 phényl-3 propyl]-L-alanyl]-glycine

Ester tert.-butylique de la N-(dihydro-2,3 indolyl-1)-N-[N-[(1S)-éthoxycarbonyl-1 phényl-3 propyl]-L-alanyl]glycine

Ester tert.-butylique de la N-(tétrahydro-1,2,3,4-isoquinolyl-2)-N-[N-[(1S)-éthoxycarbonyl-1 phényl-3 propyl]-L-alanyl]glycine

Ester tert.-butylique de la N-(imidazolyl-1)-N-[N-[(1S)-éthoxycarbonyl-1 phényl-3 propyl]-L-alanyl]-glycine

Dichlorhydrate de la N-(morpholinyl-1)-N-[N-[(1S)-éthoxycarbonyl-1 phényl-3 propyl]-L-alanyl]glycine

Ester tert.-butylique de la N-[(méthyl-2 indolidinyl-1)-1]-N-[N-[(1S)-éthoxycarbonyl-1 phényl-3 propyl]-L-alanyl]glycine

Dichlorhydrate de la N-[(méthyl-2 indolinyl-1)-1]-N-[N-[(1S)-éthoxycarbonyl-1 phényl-3 propyl]-L-alanyl]glycine

Ester tert.-butylique de la N-(pipéridino-1)-N-[N-[(1S)-éthoxycarbonyl-1 phényl-3 propyl]-L-alanyl]-glycine

Dichlorhydrate de la N-(pipéridino-1)-N-[N-[(1S)-éthoxycarbonyl-1 phényl-3 propyl]-L-alanyl]glycine

N-(N-éthylbenzylamino-N-[N-[(1S)-éthoxycarbonyl-1 phényl-3 propyl]-L-alanyl]glycine

N-(N-méthylanilino)-N-[N-[(1S)-éthoxycarbonyl-1 phényl-3 propyl]-L-lysyl]-glycine

N-(N-méthylanilino)-N-[N-[(1S)-éthoxycarbonyl-1 éthyl]-L-alanyl]glycine.

N-(diméthylamino)-N-[N-[(1S)-éthoxycarbonyl-1 phényl-3 propyl]-L-alanyl]glycine.

N-(N-méthyl-N-(pyridine-4)amino)-N-[N-[(1S)-éthoxycarbonyl-1 phényl-3 propyl]-L-alanyl]glycine.

N-(N-phénylamino)-N-[N-[(1S)-éthoxycarbonyl-1 phényl-3 propyl]-L-alanyl]glycine.

N-(N-méthylanilino)-N-[N-[(1S)-éthoxycarbonyl-1 phénoxy-2 éthyl]-L-alanyl]glycine.

Dichlorhydrate de la N-(morpholinyl-1)-N-[N-[(1S)-éthoxycarbonyl-1 phényl-3 propyl]-L-alanyl]glycine.

3. Préparation pharmaceutique comprenant au moins un composé ou un sel tel que défini à la revendication 1 ou 2, en association avec un support pharmaceutiquement acceptable.

4. Procédé de préparation de composés tels que définis à la revendication 1 ou 2, qui consiste à faire réagir, dans des conditions de formation d'amide, un composé aminé de formule (II):

$$R_5R_6N—NH—CH_2—COR_9 \qquad (II)$$

où:

$R_5$ et $R_6$ sont tels que définis à la revendication 1, et

$R_9$ représente hydrogène ou alkyle inférieur,

avec un dérivé acylant d'un acide de formule (III):

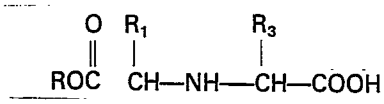

(III)

où

$R_1$ et $R_3$ sont tels que définis à la revendication 1; et

R représente hydrogène ou alkyle inférieur,

ou à faire réagir un dipeptide de formule (IV):

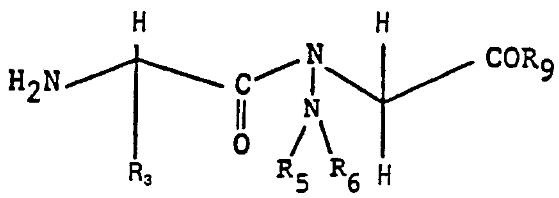

IV

où:

$R_3$, $R_5$ et $R_6$ sont tels que définis à la revendication 1; et

$R_9$ est tel que défini ci-dessus,

avec un α-céto acide ou ester de formule (V):

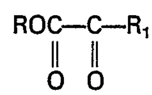

(V)

où:

$R_1$ est tel que défini à la revendication 1, et R est tel que défini ci-dessus,

pour former l'imine correspondante et à réduire ladite imine, ou à faire réagir un composé de formule VI:

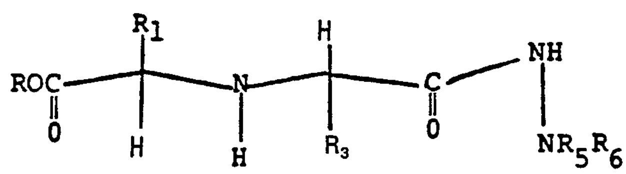

VI

où

$R_1$, $R_3$, $R_5$ et $R_6$ sont tels que définis à la revendication 1; et

R est tel que défini ci-dessus,

avec un composé α-halo de formule (VII):

$$Hal—CH_2—COR_9 \qquad (VII)$$

où:

$R_9$ est tel que défini ci-dessus; et

Hal représente halogène,

et, facultativement, à conduire des réactions de substitution ou conversion permettant d'introduire divers substituants dans lesdits produits;

et, facultativement, à former leurs sels, en particulier leurs sels pharmaceutiquement acceptables avec des acides et des bases.

**Revendications pour l'Etat contractant: AT**

1. Procédé de préparation de composés de formule générale (I):

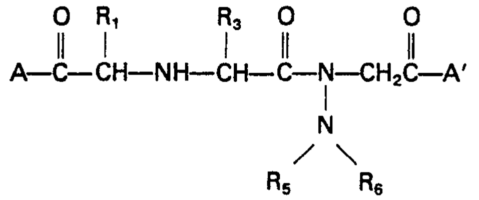

(I)

et de leurs sels pharmaceutiquement acceptables, ou:

A et A' représentent indépendamment hydroxy ou alcoxy en $C_1$—$C_6$;

$R_1$ représente alkyle en $C_1$—$C_9$, arylalkyle ou aryloxyalkyle avec 1 à 9 atomes de carbone dans la fraction alkyle; et

$R_3$ représente alkyle en $C_1$—$C_6$, ou ω-amino-alkyle en $C_1$—$C_6$,

$R_5$ et $R_6$ représente indépendamment hydrogène, alkyle en $C_1$—$C_9$, aryle, aralkyle avec 1 à 9 atomes de carbone dans la fraction alkyle, ou pyridyle, ou

$R_5$ et $R_6$, lorsqu'ils sont pris conjointement avec l'atome d'azote auquel ils sont attachés, forment un noyau monocyclique ou polycyclique, saturé ou insaturé, substitué ou non-substitué, contenant de 2 à 9 atomes de carbone, qui peut renfermer un atome d'oxygène, un atome de soufre ou un atome d'azote supplémentaire qui consiste à faire réagir dans des conditions de formation d'amide un composé aminé de formule (II):

$$R_5R_6N—NH—CH_2—COR_9 \qquad (II)$$

où:

$R_5$ et $R_6$ sont tels que définis ci-dessus et

$R_9$ représente hydrogène ou alkyle inférieur,

avec un dérivé acylant d'un acide de formule (III):

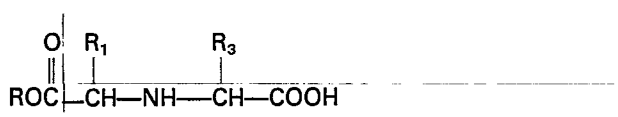

(III)

où

$R_1$ et $R_3$ sont tels que définis ci-dessus; et

R représente hydrogène ou alkyle inférieur,

ou à faire réagir un dipeptide de formule (IV):

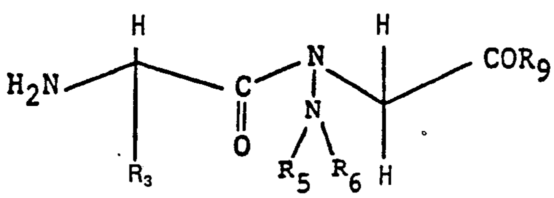

IV

où:

$R_3$, $R_5$ et $R_6$ sont tels que définis ci-dessus; et

$R_9$ est tel que défini ci-dessus,

avec un α-céto acide ou ester de formule (V):

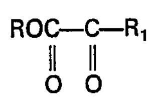

(V)

où:

$R_1$ est tel que défini ci-dessus; et

R est tel que defini ci-dessus, pour former l'imine correspondante et à réduire ladite imine, ou à faire réagir un composé de formule VI:

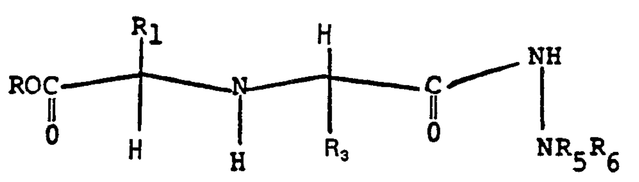

VI

où

$R_1$, $R_3$, $R_5$ et $R_6$ sont tels que définis à la revendication 1; et
R est tel que défini ci-dessus,

avec un composé α-halo de formule (VII):

$$Hal\text{-}CH_2\text{—}COR_9 \quad (VII)$$

où:

$R_9$ est tel que défini ci-dessus; et
Hal représente halogène,

et, facultativement, à conduire des réactions de substitution ou conversion permettant d'introduire divers substituants dans lesdits produits;
et, facultativement, à former leurs sels, en particulier leurs sels pharmaceutiquement acceptables avec des acides et des bases.

2. Procédé selon la revendication 1, caractérisé par le fait que l'on prépare l'un des composés suivants:

Ester tert.-butylique de la N-(N-méthylanilino)-N-[N-[(1S)-éthoxycarbonyl-1 phényl-3 propyl]-L-alanyl]-glycine
Dichlorhydrate de la N-(N-méthylanilino)-N-[N-[(1S)-éthoxycarbonyl-1 phényl-3 propyl]-L-alanyl]-glycine
Ester tert.-butylique de la N-(morpholinyl-1)-N-[N-[(1S)-éthoxycarbonyl-1 phényl-3 propyl]-L-alanyl]-glycine
Dibromhydrate de la N-[Nα-[(1S)-éthoxycarbonyl-1 phényl-3 propyl]-L-lysyl]-N-(morpholinyl-1)glycine.
Dichlorhydrate de la N-[(méthyl-2 indolinyl-1)-1]-N-[N-[(1S)-éthoxycarbonyl-1 phényl-3 propyl]-L-alanyl]glycine
Chlorhydrate de la N-[N-[(1S)-éthoxycarbonyl-1 phényl-3 propyl]-L-alanyl]-N-(pyrrolyl-1)glycine.
Ester tert.-butylique de la N-(méthyl-4 pipérazinyl-1)-N-[N-[(1S)-éthoxycarbonyl-1 phényl-3 propyl]-L-alanyl]glycine
Ester tert.-butylique de la N-(tétrahydrothiazine-1,4-yl-4)-N-[N-[(1S)-éthoxycarbonyl-1 phényl-3 propyl]-L-alanyl]glycine
Ester tert.-butylique de la N-(thiazolidinyl-3)-N-[N-[(1S)-éthoxycarbonyl-1 phényl-3 propyl]-L-alanyl]-glycine
Ester tert.-butylique de la N-(pipéridinyl-1)-N-[N-[N-[(1S)-éthoxycarbonyl-1 phényl-3 propyl]-L-alanyl]-glycine
Ester tert.-butylique de la N-(pyrrolidinyl-1)-N-[N-[(1S)-éthoxycarbonyl-1 phényl-3 propyl]-L-alanyl]-glycine
Ester tert.-butylique de la N-(dihydro-2,3 indolyl-1)-N-[N-[(1S)-éthoxycarbonyl-1 phényl-3 propyl]-L-alanyl]glycine
Ester tert.-butylique de la N-(tétrahydro-1,2,3,4-isoquinolyl-2)-N-[N-[(1S)-éthoxycarbonyl-1 phényl-3 propyl]-L-alanyl]glycine
Ester tert.-butylique de la N-(imidazolyl-1)-N-[N-[(1S)-éthoxycarbonyl-1 phényl-3 propyl]-L-alanyl]-glycine
Dichlorhydrate de la N-(morpholinyl-1)-N-[N-[(1S)-éthoxycarbonyl-1 phényl-3 propyl]-L-alanyl]glycine
Ester tert.-butylique de la N-[(méthyl-2 indolidinyl-1)-1]-N-[N-[(1S)-éthoxycarbonyl-1 phényl-3 propyl]-L-alanyl]glycine
Dichlorhydrate de la N-[(méthyl-2 indolinyl-1)-1]-N-[N-[(1S)-éthoxycarbonyl-1 phényl-3 propyl]-L-alanyl]glycine
Ester tert.-butylique de la N-(pipéridino-1)-N-[N-[(1S)-éthoxycarbonyl-1 phényl-3 propyl]-L-alanyl]-glycine
Dichlorhydrate de la N-(pipéridino-1)-N-[N-[(1S)-éthoxycarbonyl-1 phényl-3 propyl]-L-alanyl]glycine
N-(N-éthylbenzylamino-N-[N-[(1S)-éthoxycarbonyl-1 phényl-3 propyl]-L-alanyl]glycine
N-(N-méthylanilino)-N-[N-[(1S)-éthoxycarbonyl-1 phényl-3 propyl]-L-lysyl]-glycine
N-(N-méthylanilino)-N-[N-[(1S)-éthoxycarbonyl-1 éthyl]-L-alanyl]glycine.
N-(diméthylamino)-N-[N-[(1S)-éthoxycarbonyl-1 phényl-3 propyl]-L-alanyl]glycine
N-(N-méthyl-N-(pyridine-4)amino)-N-[N-[(1S)-éthoxycarbonyl-1 phényl-3 propyl]-L-alanyl]glycine
N-(N-phénylamino)-N-[N-[(1S)-éthoxycarbonyl-1 phényl-3 propyl]-L-alanyl]glycine
N-(N-méthylanilino)-N-[N-[(1S)-éthoxycarbonyl-1 phénoxy-2 éthyl]-L-alanyl]glycine
Dichlorhydrate de la N-(morpholinyl-1)-N-[N-[(1S)-éthoxycarbonyl-1 phényl-3 propyl]-L-alanyl]glycine

3. Procédé de préparation d'une composition pharmaceutique comprenant au moins un composé ou un sel tel que défini à la revendication 1 ou 2 comme ingrédient actif, consistant à formuler l'ingrédient actif avec au moins un support pharmaceutiquement acceptable et/ou au moins un diluant usuel.